(19) Europäisches Patentamt
European Patent Office
Office européen des brevets

(11) EP 0 706 567 B1

(12) **FASCICULE DE BREVET EUROPEEN**

(45) Date de publication et mention de la délivrance du brevet: **06.06.2001 Bulletin 2001/23**

(21) Numéro de dépôt: **94920523.1**

(22) Date de dépôt: **28.06.1994**

(51) Int Cl.⁷: **C12N 15/12**, C07K 14/46, A61K 38/17, C07K 16/18, C12P 21/08 // C12N15/62, C12Q1/68

(86) Numéro de dépôt international: **PCT/FR94/00780**

(87) Numéro de publication internationale: **WO 95/01431 (12.01.1995 Gazette 1995/03)**

# (54) FAMILLE DE PEPTIDES APPELES XENOXINES

PEPTIDE-FAMILIE, GENANNT XENOXINE

PEPTIDE FAMILY, DESIGNATED AS XENONINS

(84) Etats contractants désignés:
**AT BE CH DE DK ES FR GB GR IE IT LI LU MC NL PT SE**

(30) Priorité: **29.06.1993 FR 9307901**
**11.01.1994 FR 9400202**
**11.01.1994 EP 94400062**

(43) Date de publication de la demande:
**17.04.1996 Bulletin 1996/16**

(73) Titulaire: **TRANSGENE S.A.**
**67082 Strasbourg Cédex (FR)**

(72) Inventeurs:
- **KOLBE, Hanno V.J.**
  **F-67118 Geispolsheim (FR)**
- **RASMUSSEN, Ulla B.**
  **F-67118 Geispolsheim (FR)**
- **KREIL, Günther**
  **A-5081 Anif (AT)**
- **ACHSTETTER, Tilman**
  **D-77704 Oberkirch (DE)**

(74) Mandataire: **Warcoin, Jacques et al**
**Cabinet Régimbeau**
**20, rue de Chazelles**
**75847 Paris cedex 17 (FR)**

(56) Documents cités:
**EP-A- 0 607 080** **WO-A-90/13646**

- **FEBS LETTERS, vol.218, no.1, Juin 1987, AMSTERDAM NL pages 17 - 21 INOUE, S. ET AL. 'Amino acid sequence of a cytotoxin-like basic protein with low cytotoxic activity from the venom of the Thailand cobra Naja naja siamensis' cité dans la demande**
- **INVERTEBR REPROD DEV 21 (1). 1992. 15-24 REICHHART, J.-M. ET AL. 'EXPRESSION AND SECRETION IN YEAST OF ACTIVE INSECT DEFENSIN AN INDUCIBLE ANTIBACTERIAL PEPTIDE FROM THE FLESHFLY PHORMIA - TERRANOVAE.'**
- **J BIOL CHEM 268 (22). 5-08-1993. 16458-16464 KOLBE, H. ET AL. 'XENOXINS A FAMILY OF PEPTIDES FROM DORSAL GLAND SECRETION OF XENOPUS-LAEVIS RELATED TO SNAKE VENOM CYTOTOXINS AND NEUROTOXINS.' cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte à une famille de peptides, appelés xénoxines, aux membres de la famille peptidique, ainsi qu'à des procédés pour les préparer et aux séquences d'ADN correspondantes. Elle concerne également les compositions pharmaceutiques renfermant lesdites xénoxines ainsi que les trousses de diagnostic les contenant ou contenant des anticorps anti-xénoxine.

Un nombre important de peptides avec des fonctions biologiques diverses ont déjà été isolés de la peau ou des sécrétions de la peau d'amphibiens. (V. Erspamer et P. Melchiorri, Neuroendocrine Perspectives, E.E. Müller et McLeod, Eds., (Elsevier Science Publishers B.V.) *2*, (1983), p 37 ; C.L. Bevins et coll.; Ann. Rev. Biochem. *59* ; (1990) ; p 395). Dès le début on a trouvé que plusieurs de ces peptides étaient très similaires voire même identiques à des hormones et des neurotransmetteurs de mammifères (V. Erspamer et coll., Trends Pharmacol. Sci. *1*, (1980), p 391).

Ainsi la peau et les sécrétions de la peau de grenouille est réputée être une source de peptides avec des propriétés pharmacologiques ou antibiotiques intéressantes. Plus particulièrement, la peau de *Xenopus laevis*, une grenouille d'origine africaine, contient d'importantes concentrations de peptides divers.

Le rôle biologique que peuvent jouer ces peptides que l'on retrouve dans la matière sécrétée par les glandes et la peau de *Xenopus laevis* n'est que partiellement connu. D'une part, les sécrétions pourraient avoir une fonction protectrice étant donné que la matière sécrétée semblerait être nuisible aux prédateurs. D'autre part, la matière sécrétée contient des peptides qui limitent la croissance des bactéries et des champignons et qui pourraient donc se comporter comme antibiotiques sur la peau mouillée de la grenouille ou combattre les infections pendant la cicatrisation de blessures.

Ces peptides antibiotiques contiennent en général entre 21 et 26 acides aminés, sont basiques et sont exempts de tyrosine. L'identité des séquences d'acides aminés des différents peptides antibiotiques est souvent très limitée. Par contre ces peptides ont en commun leur configuration en hélice alpha amphipathique. Au niveau des séquences signal d'environ 20 acides aminés l'identité des séquences d'acides aminés n'est que de 55 % . Toutefois on retrouve des segments conservés, communs aux séquences signal des différents peptides antibiotiques. En plus, ils partagent tous une séquence N-terminale Arg-Xaa-Val-Arg qui serait le site d'action d'un enzyme de maturation commun.

Comme autres exemples de peptides isolés et caractérisés chez le *Xenopus laevis*, on peut citer la caeruléine, membre de la famille de peptides cholécystokinine/gastrine (Anastasi et coll., Brit. J. Pharmacol. *38,* (1970), p 221); spasmolysine I et II (W. Hoffmann, J. Biol. Chem. *263* (16), (1988), p 7686) ; thyrotrophine releasing peptide (K. Richter et coll., EMBO J. *3(3)*, (1984), p 617) et xénopsine (K. Araki et coll., Chem. Pharmacol. Bull. *21 (12)*, (1973), p 2801). La plupart de ces peptides sont membres d'une famille de peptides qui contient entre autres des peptides analogues d'origine mammifère. Bombésine et kassinine par exemple, ont servi comme référence pour identifier respectivement la gastrine releasing peptide et la substance K chez les mammifères (C.L. Bevins et coll, vi supra). Effectivement la matière sécrétée par la peau de grenouille contient des peptides en quantité abondante, tandis que, chez les mammifères le peptide analogue n'est souvent présent qu'en quantité faible. C'est pourquoi les peptides de grenouille se prêtent à l'identification des peptides mammifères utiles.

La présente invention concerne une nouvelle famille de peptides, que l'on a nommés xénoxines et qui possèdent des propriétés pharmaceutiques de valeur et en particulier la propriété d'influencer le fonctionnement des canaux ioniques transmembranaires tout en ne présentant pas d'activité neurotoxique. De plus, on pense que ces peptides auraient la propriété avantageuse d'éliminer les effets de l'activine.

Plus particulièrement, la présente invention se rapporte à une xénoxine caractérisée en ce qu'elle comprend une séquence d'acides aminés, laquelle :

a. contient au moins 8 cystéines (Cys) qui sont reliées par 4 ponts disulfure suivant la configuration $Cys^1$ avec $Cys^3$, $Cys^2$ avec $Cys^4$, $Cys^5$ avec $Cys^6$ et $Cys^7$ avec $Cys^8$ ;

b. contient 0 à 3 acides aminés du côté N-terminal de la $Cys^1$, 9 à 14 acides aminés entre les $Cys^1$ et $Cys^2$, 3 à 7 acides aminés entre les $Cys^2$ et $Cys^3$, 11 à 18 acides aminés entre les $Cys^3$ et $Cys^4$, 1 à 6 acides aminés entre les $Cys^4$ et $Cys^5$, 7 à 15 acides aminés entre les $Cys^5$ et $Cys^6$, aucun acide aminé entre les $Cys^6$ et $Cys^7$, 3 à 5 acides aminés entre les $Cys^7$ et $Cys^8$ et 0 à 10 acides aminés du côté C-terminal de la $Cys^8$; et

c. présente une identité d'acides aminés après alignement, d'au moins 40 % avec la séquence d'acides aminés identifiée sous le numéro SEQ ID NO: 3,

Le terme xénoxine fait référence à une petite protéine basique présentant les caractéristiques décrites.

Dans le cadre de la présente invention, le numéro figurant en indice d'un résidu cystéine indique que sa position par rapport aux autres cystéines dans ladite xénoxine dans le sens N vers C-terminal. Ainsi $Cys^1$ fait référence

à la première cystéine figurant dans une xénoxine selon l'invention du côté N-terminal.

La présente invention concerne également les peptides intermédiaires qui n'ont pas encore adopté leur conformation repliée mais qui contiennent au moins 8 cystéines, susceptibles de se relier par 4 ponts disulfure suivant la configuration $Cys^1$ avec $Cys^3$, $Cys^2$ avec $Cys^4$, $Cys^5$ avec $Cys^6$ et $Cys^7$ avec $Cys^8$.

C'est pourquoi, la présente invention se rapporte à un peptide basique caractérisé en ce qu'il comprend une séquence d'acides aminés, laquelle :

a. contient au moins 8 cystéines qui, lorsque le peptide adopte sa conformation repliée, sont reliées par 4 ponts disulfure suivant la configuration $Cys^1$ avec $Cys^3$, $Cys^2$ avec $Cys^4$, $Cys^5$ avec $Cys^6$ et $Cys^7$ avec $Cys^8$ ;

b. contient 0 à 3 acides aminés du côté N-terminal de la $Cys^1$, 9 à 14 acides aminés entre les $Cys^1$ et $Cys^2$, 3 à 7 acides aminés entre les $Cys^2$ et $Cys^3$, 11 à 18 acides aminés entre les $Cys^3$ et $Cys^4$, 1 à 6 acides aminés entre les $Cys^4$ et $Cys^5$, 7 à 15 acides aminés entre les $Cys^5$ et $Cys^6$, aucun acide aminé entre les $Cys^6$ et $Cys^7$, 3 à 5 acides aminés entre les $Cys^7$ et $Cys^8$ et 0 à 10 acides aminés du côté C-terminal de la $Cys^8$ ; et

c. présente une identité d'acides aminés après alignement, d'au moins 40 % avec la séquence d'acides aminés identifiée sous le numéro SEQ ID NO: 3,

Les peptides préférés de la présente invention contiennent 2 acides aminés du côté N-terminal de la $Cys^1$, 10 à 13 acides aminés entre les $Cys^1$ et $Cys^2$, 4 à 6 acides aminés entre les $Cys^2$ et $Cys^3$, 12 à 17 acides aminés entre les $Cys^3$ et $Cys^4$, 1 à 5 acides aminés entre les $Cys^4$ et $Cys^5$, 8 à 14 acides aminés entre les $Cys^5$ et $Cys^6$, aucun acide aminé entre les $Cys^6$ et $Cys^7$, 4 acides aminés entre les $Cys^7$ et $Cys^8$ et 2 acides aminés du côté C-terminal de la $Cys^8$.

D'autres peptides, également préférés, de la présente invention présentent une identité d'acides aminés d'au moins 50%, de manière particulièrement préférée d'au moins 60% et de manière avantageuse d'au moins 70% avec la séquence d'acides aminés identifiée sous le numéro SEQ ID NO : 3.

Les peptides particulièrement préférés conformes à l'invention sont les xénoxines caractérisées en ce qu'elles comprennent une séquence d'acides aminés, laquelle :

a. contient au moins 8 cystéines qui sont reliées par 4 ponts disulfure suivant la configuration $Cys^1$ avec $Cys^3$, $Cys^2$ avec $Cys^4$, $Cys^5$ avec $Cys^6$ et $Cys^7$ avec $Cys^8$ ;

b. contient 0 à 3 acides aminés du côté N-terminal de la $Cys^1$, 13 acides aminés entre les $Cys^1$ et $Cys^2$, 6 acides aminés entre les $Cys^2$ et $Cys^3$, 12 acides aminés entre les $Cys^3$ et $Cys^4$, 5 acides aminés entre les $Cys^4$ et $Cys^5$, 14 acides aminés entre les $Cys^5$ et $Cys^6$, aucun acide aminé entre les $Cys^6$ et $Cys^7$, 4 acides aminés entre les $Cys^7$ et $Cys^8$ et 0 à 10 acides aminés du côté C-terminal de la $Cys^8$ ; et

c. présente une identité d'acides aminés après alignement, d'au moins 80 %, de préférence 90 %, avec la séquence d'acides aminés identifiée sous le numéro SEQ ID NO : 3.

Les peptides selon l'invention comportent avantageusement de 42 à 86 acides aminés, de préférence de 51 à 75 acides aminés et, de manière particulièrement préférée, de 62 à 75 acides aminés.

La présente invention concerne également des peptides de 66 acides aminés qui présentent une identité d'acides aminés après alignement, d'au moins 80%, de manière préférée d'au moins 90% et, de manière particulièrement préférée, d'au moins 95%, avec la séquence d'acides aminés telle que identifiée sous le numéro SEQ ID NO : 3.

Comme exemples de peptides particulièrement préférés on peut citer le peptide, xénoxine-1 tel que identifié sous le numéro SEQ ID NO :3, ainsi que la xénoxine-2 selon SEQ ID NO : 4 et la xénoxine-3 selon SEQ ID NO : 5.

Pour déterminer le degré d'identité d'acides aminés, on aligne les cystéines de telle façon qu'elles se trouvent en position identique avec les cystéines de la séquence d'acides aminés telle que présentée sous SEQ ID NO: 3 et on introduit un nombre minimum d'insertions ou délétions pour maximaliser l'alignement des séquences. On identifie alors les acides aminés identiques qui se trouvent en position identique, y compris les cystéines. L'identité d'acides aminés est exprimée en pourcentage et calculée selon la formule (1) suivante :

$$\frac{n}{66 + p} \times 100 \qquad (1)$$

dans laquelle

n est le nombre d'acides aminés identiques,
66 est le nombre d'acides aminés de la séquence identifiée sous le numéro SEQ ID NO: 3,
p est le nombre d'insertions ou de délétions introduites.

Les peptides selon l'invention peuvent être sous forme de leurs sels d'addition d'acides non toxiques pharmaceutiquement acceptables. Comme exemples d'acides pharmaceutiquement acceptables, on peut citer les acides minéraux comme les acides chlorhydriques, sulfuriques, phosphoriques, etc. et les acides organiques comme les acides sulfoniques et carboxyliques tels que l'acide acétique, lactique, palmitique, stéarique, maléique, tartrique, ascorbique, citrique, etc.
Les peptides selon l'invention peuvent également être sous forme de leurs sels d'addition de base étant donné qu'ils contiennent des groupements carboxyliques libres. Comme exemples de sels pharmaceutiquement acceptables, on peut citer les sels de sodium, de potassium, de calcium ou de magnesium et les dérivés d'ammonium quaternaire.
Etant donné que les peptides selon l'invention contiennent à la fois des groupements carboxyle et amine libres, ils peuvent être sous forme de leurs sels internes ou sous forme combinée de sels d'additions et de sels internes.
Une famille de xénoxines selon l'invention comprend des peptides qui sont issus de différentes espèces d'amphibiens ou de mammifères. Comme exemple d'amphibiens on peut citer les espèces qui appartiennent au superordre des *Salienta,* anoures telles que les grenouilles, les crapauds, les rainettes, etc ; ou à l'ordre des *Caudata*, urodèles telles que les salamandres, les tritons etc. ; ou encore à l'ordre des *Gymnophiona,* apodes telles que les anguilles, les murènes etc. Il est également possible d'obtenir des xénoxines de mammifère notamment de souris, de porc ou d'homme.
Les xénoxines selon l'invention ont en commun des propriétés pharmaceutiques chez le mammifère basée notamment sur leurs interactions avec le fontionnement des canaux ioniques transmembranaires et leur interaction avec l'activine.
L'identité d'acides aminés des différentes xénoxines peut varier à cause des divergences interespèces ou alleliques. Le plus souvent il s'agit de substitutions conservatives qui permettent de conserver la conformation squelettique du peptide. Ainsi de façon préférée, dans les xénoxines selon l'invention une substitution en position homologue, comparée à la séquence identifiée sous SEQ ID NO: 3, se fait avec un acide aminé ayant une fonctionnalité équivalente pour ce qui concerne ses propriétés hydrophobiques, de charge ou d'espacement. On choisi par exemple parmi le groupe des acides aminés hydrophobes entre alanine, isoleucine, leucine, méthionine, phénylalanine, proline, tryptophane et valine ; parmi le groupe des acides aminés polaires non chargés asparagine, cystéine, glutamine, glycine, sérine, thréonine et tyrosine ; parmi les acides aminés chargés positivement arginine, histidine et lysine ; parmi les acides aminés chargés négativement acide aspartique et acide glutamique et parmi le groupe des acides aminés d'espacement équivalent alanine, glycine et sérine.
Une modification post-traductionnelle telle que la formation des ponts disulfure entre les cystéines du peptide stabilise la conformation repliée des xénoxines, ce qui permet l'exercice de leurs activités pharmacologiques.
Les différents ponts disulfure ne se forment pas de façon aléatoire. Quand on permet à la séquence d'acides aminés de s'enrouler, les groupements disulfures qui se relient sont ceux de deux cystéines voisines dans le peptide replié. C'est pourquoi la conformation du squelette peptidique peut être décrite par la configuration des ponts disulfure. A cet effet, on décrit les liaisons qui se sont formées entre les cystéines dans le peptide replié et on donne une indication du nombre d'acides aminés entre les différentes cystéines successives dans la séquence primaire des peptides.
Il existe donc une double similarité structurelle entre les peptides de la même famille, notamment au niveau du squelette peptidique, et au niveau de l'identité d'acides aminés. Ainsi, quand on compare deux peptides de la même famille, il peut se trouver qu'ils possèdent des séquences d'acides aminés avec une disparité significative, voir même une identité d'acides aminés aussi faible que 40 % . Par contre, la similitude de la configuration des cystéines impliquées dans la formation des ponts disulfure sera frappante.
Les xénoxines selon l'invention sous forme totale ou sous forme de fragment peuvent également être introduites dans des structures chimiques ou physico-chimiques plus complexes destinées à faciliter leur activité ou à leur conférer des propriétés thérapeutiques avantageuses comme par exemple la forme retard ou protégée.
L'invention concerne également les xénoxines non modifiées, par exemple non glycosylées. Cependant, l'étendue de la protection inclut également des xénoxines modifiées, par exemples des produits glycosylés, déglycosylés ou modifiés par le polyéthylèneglycol.
Les xénoxines conformes à la présente invention peuvent être isolées selon le procédé suivant :

a. on divise en fractions la matière sécrétée par la peau de grenouille par précipitation à l'aide de sulfate d'ammonium, ou un agent précipitant équivalent,

b. on soumet les précipités qui se forment entre 30 % et 70 % de saturation en sulfate d'ammonium, de préférence entre 30 % et 60 %, ou en conditions équivalentes, à une chromatographie par perméation de gel, et

c. on récolte la fraction peptidique qui élue tardivement.

Si on le désire, le mélange de xénoxines ainsi obtenu peut ensuite être purifié, notamment par HPLC d'échange de cations et suivi de HPLC phase inverse.

Selon un aspect préféré de la présente invention, la matière sécrétée par la peau de grenouille est issue de *Xenopus laevis*.

Dans le procédé selon l'invention on précipite la fraction de peptides avantageusement à l'aide de sulfate d'ammonium. Toutefois, d'autres agents précipitants peuvent être utilisés tels que le polyéthylène glycol, l'éthanol ou le sulfate de sodium. Il appartient à l'homme de métier de choisir la fourchette de saturation en agent précipitant nécessaire pour obtenir les précipités exempts d'une part de protéines et d'autre part de peptides avec des poids moléculaires inférieurs à 10 kDa, de préférence 8 kDa.

Normalement, la chromatographie par perméation de gel permet de séparer les molécules en fonction de leur taille moléculaire. Dans le procédé selon l'invention cette chromatographie permet d'écarter toutes les molécules de taille moléculaire importante, des peptides concernés en récoltant uniquement la fraction qui élue tardivement de la colonne.

De ce fait chaque type de gel de perméation ayant une zone de fractionnement au delà de 10 kDa peut être utilisé. Dans le commerce ce genre de gel est disponible sous le nom, par exemple, de Sephacryl S200 (Pharmacia), Sephadex (Pharmacia), Fractogel (Merck) ou Toyopearl (Toso Haas).

On peut également synthétiser chimiquement les peptides selon l'invention par des méthodes de synthèse connues de l'homme du métier. A titre de référence on citera E. Bayer, Angew. Chem. Int. Ed. Engl. *30*, (1991), p 113.

La présente invention concerne également l'expression des xénoxines dans des systèmes cellulaires autres que le système cellulaire naturel dont elles sont issues.

Pour ce faire, l'invention concerne également les séquences d'ADN isolées codant pour lesdites xénoxines, ainsi que des cassettes d'expression desdites séquences assurant leur expression dans la cellule désirée.

Par fragment d'ADN isolé, on entend un fragment d'ADN qui n'est plus associé à l'ensemble des régions naturelles qui contrôlent son expression dans l'organisme dont il est issu, c'est à dire dans le cas de l'exemple cité ci-dessous du *Xénopus laevis*.

Parmi les séquences d'ADN isolé qui comprennent une séquence codant pour un peptide selon l'invention, il faut citer en particulier l'ADN qui est repris dans la liste d'identification des séquences sous le numéro SEQ ID NO: 1, et plus particulièrement la partie de la SEQ ID NO: 1 de la position 39 à 290 et la partie de la SEQ ID NO: 1 de la position 93 à 290.

L'invention concerne des cassettes d'expression desdites séquences d'ADN codant pour un peptide selon l'invention.

Une telle cassette d'expression comprend notamment un fragment d'ADN isolé qui est placé sous le contrôle d'éléments permettant sa transcription et sa traduction dans la cellule hôte considérée.

Ces éléments de contrôle sont essentiellement un promoteur de la transcription approprié ainsi que des codons d'initiation et de fin de traduction. Dans certains cas, il est aussi avantageux d'ajouter un terminateur de transcription et un peptide signal, ou séquence pré, éventuellement suivie d'une séquence pro. On préférera, tout particulièrement, la séquence pro de la défensine A de *Phormia terranovae* (Dimarcq et coll., EMBO J, *9* (1990) p. 2507) fonctionnelle dans les cellules eucaryotes et notamment la levure comme *Saccharomyces cerevisiae.*

L'invention concerne également une cellule qui est transformée avec une cassette d'expression qui comprend un fragment d'ADN selon l'invention. La cassette d'expression peut être soit intégrée dans le génome de la cellule soit portée par un vecteur d'expression approprié tel que par exemple un plasmide ou un vecteur viral.

La présente invention concerne également l'utilisation de vecteurs d'expression susceptibles d'être administrés pour produire les xénoxines *in situ* chez l'homme ou l'animal, notamment lorsque le vecteur est un vecteur viral ou synthétique (par exemple des mélanges de lipides comme les liposomes).

Enfin, la présente invention concerne un procédé de préparation d'un peptide selon l'invention qui consiste à cultiver une cellule transformée selon l'invention et à récolter ledit peptide à partir de la culture.

Les technologies permettant le clonage et l'expression d'un gène étranger dans des cellules hôtes procaryotes ou eucaryotes sont connues de l'homme du métier. Elles seront illustrées dans les exemples ci-dessous, étant entendu que d'autres vecteurs et d'autres cellules hôtes peuvent être utilisés. L'homme du métier sait bien évidemment choisir le promoteur de la transcription approprié en fonction de l'hôte dans lequel on souhaite exprimer le fragment d'ADN et en fonction du vecteur dans lequel la cassette d'expression doit être insérée.

Selon un procédé préféré de préparation d'un peptide selon l'invention le fragment d'ADN isolé est exprimé dans des cellules hôtes qui permettent la sécrétion des peptides synthétisés dans le milieu de culture et d'assurer la formation des ponts disulfure. A titre d'exemple, on peut citer les cellules hôtes de procaryotes tel que *E. coli*, d'eucaryotes inférieurs tel que *Saccharomyces cerevisiae* ou de eucaryotes supérieurs tel que l'homme ou l'animal ou les plantes.

Pour diriger un peptide selon l'invention vers le système de sécrétion des cellules hôtes, on prévoit dans la cassette d'expression une séquence codant pour un précurseur de celui-ci. Ledit précurseur comprend essentiellement un peptide selon l'invention sous forme mature et un peptide signal fixé par liaison peptidique à son extrémité NH2 terminale. Un peptide signal comporte généralement une séquence d'acides aminés principalement hydrophobe et a pour fonction de promouvoir la sécrétion. Pendant le processus de translocation ce peptide signal sera clivé par un enzyme, la peptidase signal. C'est alors un peptide mature qui est sécrété dans le milieu de culture ou dans le périplasme de l'hôte.

Le peptide signal préférablement utilisé est issu de gènes dont il est connu qu'ils codent pour un produit sécrété de façon efficace. A titre d'exemple on cite les peptides signal de l'invertase périplasmique SUC2 (R.A. Smith et coll., Science *229*, (1985), p 1219; C.N. Chang et coll., Mol. Cell. Biol. *6*, (1986), p 1812), ainsi que les peptides signal toxines "killer" de *Kluyveromyces lactis* (C. Baldazi et coll., EMBO J. *6*, (1987), p 229) ou de *Saccharomyces cerevisiae* (M. Tokunaga et coll., Nucleic Acids Res. *16*, (1988), p 7499). A titre d'indication, on précise toutefois que le peptide signal du gène *BGL2* issu de *S. cerevisiae* (T. Achstetter et coll., Gene *110,* (1992) p 25), et utilisé dans les exemples ci-dessous, permet d'obtenir de très bons résultats. Par ailleurs, on peut également employer le peptide signal naturel des xénoxines.

Dans certains cas, le peptide obtenu pourra ne pas présenter la bonne configuration, il pourra alors être nécessaire de procéder à son réarrangement par des méthodes chimiques comportant, par exemple, la mise en solution et repliement en utilisant des conditions de pH et l'urée par exemple.

La présente invention concerne également les anticorps monoclonaux ou polyclonaux anti-xénoxines qui peuvent être obtenus par des procédés connus ainsi que leur application tant en thérapie que dans le domaine diagnostic pour identifier ou quantifier les xénoxines chez l'homme ou chez l'animal. Le choix de la technique à mettre en oeuvre est large et à la portée de l'homme du métier. On peut citer notamment les techniques ELISA, de marquage ou encore de fluorescence.

La présente invention concerne donc également des trousses de diagnostic utilisant les xénoxines ou les anticorps correspondants.

Les peptides selon l'invention ont des applications thérapeutiques chez les mammifères et notamment chez l'homme étant donné leurs propriétés de pouvoir influencer le fonctionnement des canaux ioniques transmembranaires, et de l'activine, sans avoir d'activité neurotoxique.

En conséquence l'invention se rapporte également à :

- une composition pharmaceutique qui comprend à titre d'agent actif au moins un peptide selon l'invention ;
- l'usage thérapeutique d'un peptide selon l'invention ;

La composition pharmaceutique selon l'invention est en particulier destinée au traitement préventif ou curatif de maladies telles que par exemple des arythmies cardiaques dues à une hyper ou hypotension ; des maladies fibrokystiques d'origine génétique telle que la fibrose kystique du pancréas, mieux connue sous le nom de mucoviscidose ; les troubles associés à un déséquilibre de gonadotrophine chorionique humaine pendant la grossesse ; les maladies dues à une sécrétion déséquilibrée des hormones FSH, STH et ACTH (respectivement, pour follicle stimulating hormone, somatotropic hormone et adenocorticotropic hormone en Anglais) ; les compositions pharmaceutiques selon l'invention permettent également le réglage de l'érythropoièse.

Les compositions pharmaceutiques renfermant les peptides selon l'invention peuvent être administrées par la voie la plus appropriée à sa destination finale, notamment par voie orale, parentérale ou rectale.

Les compositions pharmaceutiques utilisables pour l'administration orale peuvent être solides ou liquides, par exemple sous forme de comprimés (enrobés ou non), pilules, dragées, capsules en gélatine, solutions, sirops, etc.

De même, les compositions utilisables pour l'administration par voie parentérale, sont les formes pharmaceutiquement connues pour ce genre d'administration, par exemple des solutions, des suspensions ou émulsions aqueuses ou huileuses, qui conviennent e.a. pour des injections intramusculaires, intraveineuses, intrapéritonéales, sous cutanées ou des applications cutanées ou transmuqueuse, par exemple par spray, pommades etc.

Pour l'administration par voie rectale, les compositions contenant les composés de l'invention se présentent généralement sous forme de suppositoires.

Les composés selon l'invention pourront également être préparés sous une forme à libération contrôlée et, en fonction de la voie d'administration retenue, sous une forme protégée appropriée afin d'éviter une dégradation trop rapide du peptide.

Les formes pharmaceutiques telles que solutions injectables, suspensions injectables, comprimés, gouttes, suppositoires, etc. sont préparées selon les méthodes couramment utilisées par les pharmaciens. Les formes pharmaceutiques comprennent également les compositions qui permettent de délivrer le peptide actif d'une manière progressive. On mélange les composés de l'invention avec un véhicule solide ou liquide, non toxique, pharmaceutiquement acceptable, et éventuellement avec un agent dispersant, un agent désintégrant, un agent stabilisant etc. On peut y ajouter, par exemple, des agents édulcorants, des agents colorants, etc. Le pourcentage de produit actif dans les compositions pharmaceutiques peut varier dans des limites très larges, selon le patient et le mode d'administration, en particulier selon la fréquence d'administration.

Les exemples qui suivent illustrent la présente invention, sans la limiter, il y est fait référence aux figures 1 à 4.

La figure 1 représente un SDS-PAGE coloré au Bleu de Coomassie de deux stades différents de purification. (1) fraction peptidique récoltée après chromatographie par perméation de gel Sephacryl S300 ; (2) Marqueurs de poids moléculaire d'en bas jusqu'en haut : lysozyme (14300), inhibiteur de trypsine (21500), anhydrase carbonique (30000), ovalbumine (46000), albumine de sérum bovin (69000) phosphorylase b (92500) et myosine (200000) ; (3) xénoxine-1 purifiée par HPLC d'échange de cations et phase inverse selon l'exemple 1 ci-dessous.

La figure 2 représente un profil d'élution obtenu après HPLC à échange de cations de la fraction peptidique issue de sécrétion de peau de *Xenopus laevis* obtenue par précipitation au sulfate d'ammonium suivie d'une chromatographie par perméation de gel dans les conditions de l'exemple 1 qui est repris ci-dessous. Les fractions qui contiennent respectivement xénoxine-1, xénoxine-3 et xénoxine-2 sont indiquées par flèches successives.

La figure 3 A représente l'alignement de (a) xénoxine-1 avec (b) la cytotoxine issue de *Naja naja kaouthia, Naja naja siamensis*, un cobra monocle (S. Inoue et coll., FEBS Lett. *218,* (1987), p 17) et (c) la neurotoxine d courte de *Laticauda colubrina*, un bungare de mer aux lèvres jaunes (N. Tamiya et coll. Toxicon (Suppl. 3), (1983), p 445). Des écartements (-) sont introduits et les ponts disulfure indiqués.

La figure 3 B représente le nombre d'acides aminés entre les cystéines successives dans les séquences primaires pour les trois familles de peptides xénoxines, cytotoxines et neurotoxines courtes, respectivement.

La figure 4 représente l'alignement de xénoxine-1 avec 27 acides aminés du site de fixation N'-terminal de 116 résidus du récepteur humain de l'activine.

**Exemple 1 : Purification et caractérisation des xénoxines.**

**1.1 Isolement et purification des xénoxines.**

*Xenopus laevis* (Herpetologic Institute, DeRover, Pays Bas) est maintenu dans des bacs aérés et alimentés avec du foie de porc haché. Toutes les trois semaines, les animaux sont soumis à un léger choc électrique (15 V) et les sécrétions de leur peau sont récoltées dans une solution de 0,9 % de NaCl selon la méthode décrite par C. Mollay et coll., Eur. J. Biochem. *160,* (1986), p 31. Le mucus ainsi extrait est soumis à deux extractions successives par le n-butanol à volume égal. La fraction insoluble est éliminée par centrifugation pendant 15 minutes à 15.000 tours par minutes (Sorvall RC-5B, rotor SS34). Du sulfate d'ammonium est ensuite ajouté au surnageant et le matériel précipitant entre 30 et 60 % de saturation est récupéré. Ce précipité est dissous et dialysé dans l'acétate d'ammonium 50 mM, pH 5,0 et soumis à une chromatographie par perméation de gel en utilisant une colonne Sephacryl S300 (2,7 cm x 120 cm ; Pharmacia) équilibrée avec le même tampon. La fraction peptidique est détectée à 280 nm et élue tardivement sous forme d'un pic large. Seule cette fraction tardive est collectée et lyophilisée.

On établit un SDS-PAGE coloré au Bleu de Coomassie pour vérifier que les molécules de poids moléculaire supérieur à 10 kDa sont écartées, (voir Figure 1).

La fraction peptidique lyophilisée est reprise dans 50 ml d'acétonitrile 5 %, dans l'acétate d'ammonium 20 mM à pH 4,6 puis centrifugée pendant 15 minutes à 15.000 tours par minute (Sorvall RC-5B, rotor SS34). Le surnageant est filtré sur filtre Millex GV de 0,22 µm (Millipore) et des aliquotes de 5ml sont congelées à -80°C.

Une aliquote de 5 ml de la solution est diluée dans 90 ml de tampon CE1 (acétonitrile 25 % dans du phosphate de potassium monobasique 5 mM ajusté à pH 3,0 à l'aide de l'acide phosphorique). La conductivité de la solution est ajustée à 8 mS à l'aide du tampon CE1 et si nécessaire, le pH est ajusté à 3,0 à l'aide de l'acide phosphorique. La solution ainsi obtenue est alors séparée en trois aliquotes qui sont successivement soumises à une séparation par HPLC (Chromatographie Liquide Haute-Performance) d'échange de cations avec détection UV à 215 nm, de la façon suivante :

- l'aliquote est chargée sur une colonne HPLC (poly-Sulfoéthyle Aspartamide SCX ; 9,4 mm x 200 mm ; The Nest Group) à un débit de 1,6 ml/minutes ;
- la colonne est lavée avec le tampon CE1 jusqu'à ce que la ligne de base à 215 nm se soit stabilisée ;
- un gradient 0 à 100 % de tampon CE2 (chlorure de potassium 600 mM dans du tampon CE1) est appliqué en 40 minutes, pour éluer le matériel adsorbé qui est collecté manuellement par fractions de 1,6 ml ;
- la colonne est lavée par le tampon CE2 pendant 10 minutes ;
- un gradient 100 % tampon CE2 à 100 % de tampon CE1 est appliqué en 5 minutes ; et
- la colonne est lavée par le tampon CE1 pendant 15 minutes.

On obtient respectivement la xénoxine-1 qui élue entre 362 et 392 mM de chlorure de potassium après 33,3 à 35,5 minutes ; la xénoxine-3 (398-415 mM; 35,9-37,1 minutes) ; et la xénoxine-2 (415-435 mM ; 37,1-38,6 minutes) qui sont indiquées par les flèches successives sur le diagramme de la Figure 2.

Une étape de séparation en HPLC phase inverse (Hewlett Packard 1090A) avec détection UV à 205 nm est ensuite réalisée. A cet effet, les fractions individuelles issues de la chromatographie HPLC d'échange de cations sont directement chargées sur une colonne HPLC Vydac $C_{18}$ (10 mm x 250 mm ; Vydac Separation Group) équilibrée avec le tampon RP1 (0,10 % (v/v) d'acide trifluoroacétique (TFA) dans l'eau dé-ionisée par le système de préparation d'eau réactif de Millipore (eau Milli Q)) à un débit de 1,5 ml/minute.

Pour chaque fraction chargée, les xénoxines sont éluées de la colonne selon la procédure suivante :

- 100 % tampon RP1 pendant 5 minutes pour restabiliser la colonne ;
- un gradient 100 % de tampon RP1 à 71,4 % de tampon RP2 (30/70/0,10 (v/v/v) d'eau Milli Q/acétonitrile/TFA) est appliqué en 100 minutes ;
- la colonne est lavée par 71,4 % de tampon RP2 pendant 10 minutes ;
- un gradient 71,4 % de tampon RP2 à 100 % de tampon RP1 est appliqué en 5 minutes ; et
- la colonne est lavée par 100 % de tampon RP1 pendant 15 minutes.

Les fractions sont collectées manuellement et les xénoxines désalées et purifiées sont concentrées à l'aide d'un Speed Vac Concentrator (Savant) et stockées à -20°C.

On obtient ainsi, à partir d'un équivalent de sécrétions de peau de 150 *Xenopi laevis*, 800 µg de xénoxine-1, 650 µg de xénoxine-2 et 200 µg de xénoxine-3.

**1.2 Caractérisation des xénoxines**

**1.2.1 Réduction et alkylation**

50µg de xénoxines purifiées et concentrées selon l'exemple 1.1 sont repris dans un tampon d'alkylation (Tris-HCl 250 mM, pH 8,5 ; chlorure de guanidinium 6M ; EDTA 1 mM) à une concentration de 250µg/ml, puis homogénéisées au vortex et soniquées 5 minutes. Ensuite 5µl de 20/80 (v/v) β-mercaptoéthanol en eau Milli Q sont ajoutés, le mélange est mis sous argon et laissé à 37°C. Après 2 heures, 10µl de 4-vinylpyridine (4VP ; Janssen) sont ajoutés, le mélange est à nouveau mis sous argon et laissé à température ambiante. Après 2 heures, le mélange de réaction est congelé et gardé à -20°C jusqu'à son utilisation pour digestion ou désalage.

**1.2.2 Digestion à la clostripaïne (CL), trypsine (T) ou au bromure de cyanogène (CNBr).**

La clostripaïne (Sigma) est activée à une concentration de 1 mg/ml dans du tampon CL (Tris-HCl 100mM, pH 7,5 ; dichlorure de calcium 1mM, DTT 2,5 mM) à 37°C pendant 2 heures. 12 µg de xénoxine préparée selon l'exemple 1.1. (alkylée avec 4VP, selon l'exemple 1.2.1, ou non-alkylée), sont incubés dans le tampon CL pendant 16 heures à 37°C à une concentration de 150 µg/ml et un rapport en poids protéase/substrat de 1:10. 8 µl d'acide acétique glacial et 70µl du tampon RP1 (voir ci-dessus) sont ensuite ajoutés. La solution ainsi obtenue est alors utilisée pour déterminer la carte peptidique par chromatographie HPLC phase inverse comme décrit dans l'exemple 1.2.3, ci-dessous.

La trypsine (1 mg/ml dans HCl 1mM) (Boehringer) est diluée 10 fois dans du tampon TR (N-éthylmorpholine-HCl 100mM, pH 8,3 ; dichlorure de calcium 0,1 mM). 25 µg de xénoxine préparée selon l'exemple 1.1. (alkylée avec 4VP ou non-alkylée), sont incubés dans le tampon TR pendant 16 heures à 37°C à une concentration de 240 µg/ml et un rapport en poids protéase/substrat entre 1:5 et 1:100. 2µl de tampon TFA pur et 100 µl de tampon RP1 sont ensuite ajoutés et la solution ainsi obtenue est utilisée pour déterminer la carte peptidique par chromatographie HPLC phase inverse comme décrit dans l'exemple 1.2.3.

40 µl d'une solution (poids/volume) CNBr 1% (Pierce) dans de l'acide formique 70 % sont ajoutés à 25 µg de xénoxine préparée selon l'exemple 1.1. (alkylée avec 4VP ou non-alkylée). La réaction se poursuit pendant 16 heures après addition de 300 µl d'eau Milli Q. Le mélange de réaction est séché à l'aide d'un Speed Vac Concentrator. 250 µl de tampon RP1 sont ajoutés. La solution ainsi obtenue est utilisée pour déterminer la carte peptidique par chromatographie HPLC phase inverse comme décrit dans l'exemple 1.2.3.

**1.2.3. Détermination de la carte peptidique par chromatographie HPLC phase inverse.**

La carte peptidique est réalisée par chromatographie HPLC phase inverse à l'aide d'une unité HPLC 1090A (Hewlett-Packard) avec détection UV à 205 nm.

Les molécules de xénoxine intactes, leurs fragments protéolytiques ainsi que leurs équivalents alkylés avec 4VP sont chargés sur une colonne HPLC Superspher 100$C_{18}$ (4 mm x 125mm ; Merck) équilibrée avec TFA 10 mM dans 10/90 (v/v). acétonitrile/eau Milli Q à un débit de 1 ml/minute. Les peptides sont alors élués suivant le protocole suivant :

- La colonne est lavée avec TFA 10 mM dans 10/90 (v/v) acétonitrile/eau Milli Q pendant 15 minutes ; et

- un gradient linéaire jusqu'à TFA 10 mM dans 50/50 (v/v) acétonitrile/eau Milli Q est appliqué en 40 minutes.

Les fractions éluées sont collectées manuellement et concentrées à l'aide d'un Speed Vac Concentrator.
Les temps de rétention observés sont repris dans les tableaux I, II et III ci-dessous.

**1.2.4. Détermination de la séquence en acides aminés.**

Les peptides obtenus selon l'exemple 1.2.3. sont solubilisés dans une solution d'acide formique 80 % et la détermination de la séquence N-terminale est réalisée à l'aide d'un séquenceur de protéines de type 477A couplé à un analyseur HPLC d'acides aminés phénylthiocarbamique (Applied Biosystems Inc.) selon les procédures standards. Les résultats du séquençage sont présentés dans les tableaux I, II et III ci-après.

**TABLEAU I**

| Peptide (1) | temps de rétention (minutes) | SEQUENCE EN ACIDES AMINES (10 20 30 40 50 60) |
|---|---|---|
| xénoxine-1, alkylée[a] | 37,5 | LKCVNLQANGIKMTQECAKEDTKCLTLRSLKKTLKFC |
| xénoxine-1, alkylée fragment CNBR1[b,c,d] | 34,0 | LKCVNLQANGIKXXQECAKEDTKCLTLRSLKKTLKFCAXXXTC<br>TQECAKEDTKCLTXRSLKKTLKFCAXGRTCT |
| xénoxine-1, alkylée fragment CL1[b] | 27,7 | LKCVNLQA<br>SLKKTLKFCASG |
| xénoxine-1 fragment T1[b,d] | 33,4 | TXTTMK<br>IMSLPGEQITXXEGNMXNA |
| xénoxine-1, alkylée fragment CNBR2[b,c] | 28,2 | SLPGEQITCCEGN<br>KIMSLPGEQITCCEGN |
| xénoxine-1, alkylée fragment CL2 | 32,9 | IMSLPGEQITCCEGNMCN |
| xénoxine-1 | 40,0 | LKCVNLQANGIKMTQECAKEDTKCLTLRSLKKTLKFCASGRTCTTMKIMSLPGEQITCCEGNMCNA |

(1) CNBR, CL ou T précisent la méthode protéolitique utilisée
a Molécule complète, seule la partie analysée est présentée
b Deux séquences peptidiques sont lues en parallèle
c Un peptide dérive d'un clivage incomplet
d Les résidus marqués X ne sont pas identifiables

**TABLEAU II**

| Peptide (1) | temps de rétention (minutes) | SEQUENCE EN ACIDES AMINES<br>10 20 30 40 50 60 |
|---|---|---|
| xénoxine-2, alkylée[a] | 37,5 | LKCVNLQANGIKMTQECAKEDNKCLTLRSLKKTLKFCAXDRICKTMKIMSLPGEXI |
| xénoxine-2[a,b] | 40,0 | LKXVNLQANGIKMTQEXAKEDNKXLTLRSLKKTLKFCASDXIXKTMK |
| xénoxine-2, alkylée fragment CL3 | 23,0 | ITCCEGNMCNA |
| xénoxine-2 fragment T3 | 28,0 | IMSLPGEK |
| xénoxine-2 | 40,0 | LKCVNLQANGIKMTQECAKEDNKCLTLRSLKKTLKFCASDRICKTMKIMSLPGEKITCCEGNMCNA |

(1) CL ou T précisent la méthode protéolitique utilisée
a Molécule complète, seule la partie analysée est présentée
b Les résidus marqués X ne sont pas identifiables

## TABLEAU III

| Peptide (1) | temps de rétention (minutes) | SEQUENCE EN ACIDES AMINES (10 · 20 · 30 · 40 · 50 · 60) |
|---|---|---|
| xénoxine-3, alkylée[a,b,c] | 35,5 | LXCVNLQANGXKMXQECXKE<br>KCVNLQANGVKMTQECAKED<br>CVNLQANGVKMTQECAKEDT |
| xénoxine-3, alkylée fragment T4 | 7,0 | FCASDR |
| xénoxine-3, alkylée fragment T5 | ? | IASLPGEQITCCEGNMCNA |
| xénoxine-3, alkylée fragment T6[b] | ? | IASLPGEQITXXEGNMXNA |
| xénoxine-3[d] | 40,0 | LKCVNLQANGVKMTQECAKEDT*KCLTLRSLKKTLK*FCASDR*ICKTMK*IASLPGEQITCCEGNMCNA |

(1) T précise la méthode protéolitique utilisée
a Molécule complète, seule la partie analysée est présentée
b Les résidus marqués X ne sont pas identifiables
c Trois séquences peptidiques sont lues en parallèle
d Les acides aminés en italiques sont déduits des séquences des xénoxine-1 et xénoxine-2

### 1.2.5 Spectrométrie de masse

Les mesures de spectrométrie de masse sont effectuées par ESMS (Electrospray Mass Spectrometry) sur un spectromètre de masse de type VG Bio Tech BioQ (VG Biotech Ltd.). Les protocoles utilisés sont connus de l'homme du métier et sont repris par exemple dans A. Van Dorsselaer et coll., Biomed. Environ. Mass Spectrom. *19*, (1990), p 692. Les masses moléculaires calculées sont données comme des valeurs moyennes basées sur les valeurs des masses atomiques des éléments C, H, N, O et S suivantes : C = 12,011 ; H = 1,0079 ; N = 14,0067 ; O = 15,9994 et S = 32,06.

Les résultats sont présentés dans le tableau IV ci-après.

TABLEAU IV

| SPECTROMETRIE DE MASSE | | |
|---|---|---|
| Peptide | Masse moléculaire expérimentale | Masse moléculaire calculée |
| xénoxine-1 | 7228,3 ±0,1 Da[a] | 7227,6 Da[b] |
| xénoxine-1, alkylée | 8077,7 ± 0,4 Da[c] | 8076,7 Da[d] |
| xénoxine-1, T1 | 2693,5 ± 0,2 Da | 2694,6 Da[e] |
| xénoxine-1, T2 | 1662,0 ± 0,2 Da | 1662,0 Da[f] |
| mélange de fragments | 2329,2 ± 0,6 Da | 2329,7 Da[g] |
| trypsiques | 1164,5 ± 0,4 Da | 1164,3 Da[h] |
| xénoxine-1, alkylée | 744,4 Da | 744,8 Da[i] |
| | 709,4 Da | 709,5 Da[j] |
| xénoxine-2 | 7337,5 ± 0,5 Da | 7337,8 Da[b] |
| xénoxine-2, alkylée | 8188,7 ±0,5 Da[c] | 8186,9 Da[d] |
| xénoxine-2, fragment T2' | 1661,9 ± 0,6 Da | 1662,0 Da[f] |
| mélange de fragments | 1473,0 ± 0,2 Da | 1473,7 Da[k] |
| trypsiques | 1164,7 ± 0,2 Da | 1164,3 Da[h] |
| xénoxines-2, alkylée | 873,5 Da | 874,1 Da[l] |
| | 709,5 Da | 709,5 Da[j] |
| xénoxine-3 | 7251,2 ± 0,4 Da | 7250,6 Da[b] |
| xénoxine-3, alkylée | 8100,6 ±0,8 Da[c] | 899,8 Da[d] |

a Masse correspondant au signal majoritaire ; un signal moins intense donne une masse de 7243,8 ± 0,4 Da, probablement dû à l'addition d'un atome d'oxygène à la première méthionine.

b En admettant que les 4 ponts disulfure soient intacts.

c Indiquant 8 composés 4VP par protéine ; pour la xénoxine-1 un signal moins intense donne une masse de 8091,8 ± 0,9 Da, probablement dû à l'addition d'un atome d'oxygène.

d Xénoxine réduite, alkylée avec 8 composants 4VP.

e TCTTMK, cystéine liée à IMSLPGEQITCCEGNMCNA ; en admettant que les 2 ponts disulfure soient intacts.

f CVNLQANGIK, cystéine liée à CLTLR, en admettant que le pont disulfure soit intact.

g IMSLPGEQITCCEGNMCNA, alkylé par 3 composés 4VP.

h CVNLQANGIK, alkylé par un composé 4VP.

i FCASGR, alkylé par un composé 4VP.

j CLTLR, alkylé par un composé 4VP.

k ITCCEGNMCNA, alkylé par 3 composés 4VP.

l IMSLPGEK.

### Exemple 2 : Clonage de l'ADN complémentaire codant pour des xénoxines

Pour plus de précisions sur les techniques générales de manipulation des acides nucléiques et de clonage moléculaire qui ne sont pas détaillées dans l'exemple qui suit, on peut se reporter à l'ouvrage de Maniatis et coll. (Laboratory Manual, Cold Spring Harbor Laboratory Press, Cold Spring Harbor, NY (1989)).

### 2.1. Synthèse des amorces oligonucléotidiques

En utilisant un synthétiseur d'oligonucléotides ABI 380B (Applied Biosystems Inc.) suivant les procédures standards préconisées par le constructeur, 0,2 μmole des oligonucléotides suivants sont synthétisés. Les bases dégénérées sont exprimées selon leur code à une lettre de l' "International Union of Biochemistry" (I.U.B.) ; I correspond à l'inosine.

OTG3278 (5'-ATGTCGACCARGARTGYGCIAARGARGA-3'), *i.e.* brin sens-Q-E-C-A-K-E-D avec addition d'un site de restriction *Sal*I à l'extrémité 5' afin de faciliter le sous clonage ultérieur (SEQ ID NO:26) ; et

OTG3279 (5'-ATAAGCTTCACATRTTICCYTCRCARCA-3'), *i.e.* brin antisens-C-C-E-G-N-M-C avec addition d'un site *Hind*III à l'extrémité 5' afin de faciliter le sous clonage ultérieur (SEQ ID NO: 27).

Après déprotection, les oligonucléotides sont purifiés sur μ-Bondapak $C_{18}$ (3,5 mm x 300mm ; Waters) à l'aide d'un gradient 20% - 30% d'acétonitrile en présence de triéthylamine/acide acétique 100mM, pH 6,9. Les oligonucléotides sont détritylés suivant les conditions standard bien connues par l'homme du métier et quantifiés à l'aide de leur spectre UV entre 230 et 340 nm.

### 2.2. Amplification génique

De l'ARN messager de *Xenopus laevis* est obtenu selon la méthode décrite dans K. Richter, et coll, J. Biol. Chem. *261,* (1986), p 3676 et est alors soumis à une transcription inverse initiée par des amorces dégénérées. On obtient ainsi l'ADNc qui servira de matrice pour l'amplification. Une étape d'amplification génique par PCR (Polymerase Chain Reaction) est alors réalisée sur 0,5 μg de l'ADNc dans 50 μl contenant 250 ng des oligonucléotides OTG3278 (brin sens) et OTG3279 (brin anti-sens) synthétisés selon l'exemple 2.1, ainsi que Tris-HCl 10 mM, pH 8,3 ; chlorure de potassium 10mM ; dichlorure de magnésium 1,6 mM ; DTT 1 mM ; 200μM de chacun des désoxyribonucléosides triphosphates (dATP, dCTP, dGTP et dTTP) (Pharmacia) et 2,5 unités d'ADN Polymérase AmpliTaq (Perkin Elmer Cetus). Une unité étant la quantité d'enzyme qui permet la synthèse de 10 nmoles d'ADN pendant 30 minutes d'incubation. 30 cycles d'amplification sont réalisés selon le protocole suivant :

Lors des 5 premiers cycles :

- Dénaturation à 93°C pendant 1 minute (3 minutes lors du premier cycle) ;
- hybridation des amorces à 50°C pendant 2 minutes ;
- polymérisation effectuée à 72°C pendant 30 secondes, avec une augmentation de température de 1°C par 20 secondes ;

Lors des 25 cycles restants :

- dénaturation à 93°C pendant 1 minute ;
- hybridation à 60 °C pendant 1 minute ; et
- polymérisation à 72°C pendant 45 secondes.

### 2.3. Analyse

En électrophorèse sur gel d'agarose (1%) une aliquote de 5μl du produit de PCR présente une large bande d'environ 120 paires de base.

### 2.4. Clonage de l'ADNc-PCR

L'ADN restant est soumis à une digestion à l'aide des enzymes de restriction *Hind*III et *Sal*I, (Gibco-BRL) puis les fragments d'ADN sont séparés selon leur taille à l'aide d'agarose à point de fusion bas (agarose type II Sigma). Les ADN ainsi séparés sont purifiés et clonés dans un bactériophage M13 selon les procédures standards. Les fragments d'ADNc-PCR ainsi clonés sont utilisés comme sonde pour le criblage de la banque d'ADNc de peau de *Xenopus laevis*.

### 2.5. Criblage de la banque d'ADNc de peau de *Xenopus laevis.*

On prépare une sonde marquée au $^{32}P$ en utilisant le fragment d'ADNc-PCR, PCR1, d'un clone préparé selon l'exemple 2.4. La séquence de l'insert PCR1 est représentée dans la liste des séquences sous le numéro SEQ ID NO: 28. PCR1 est marquée avec $\alpha[^{32}P]$dATP (Amersham) selon la méthode des amorces aléatoires (Boehringer Mannheim).

Pour le criblage, on utilise la banque d'ADNc de la peau de *Xenopus laevis* construite dans le vecteur d'expression λgt11, selon Kuchler et coll., J. Biol. Chem. *265*, (1990), p 11731. Des boîtes contenant environ 2 x $10^4$ plages de phage sont préparées et une empreinte est réalisée sur filtre de nitrocellulose BA85 (Schleicher & Schüll).

Les filtres empreintés sont alors hybridisés avec la sonde PCR1 $^{32}P$ dans 2 x SET (chlorure de sodium-EDTA-Tris-HCl), 10 x solution de Denhardt et SDS 0,1% à 55°C.
Les clones positifs sont utilisés dans deux criblages consécutifs.

### 2.6. Séquençage des clones d'ADNc

L'ADN des phages positifs est isolé et les inserts d'ADNc sont sous-clonés dans des vecteurs M13TG130 (Kieny et coll., Gene *26*, (1983), p 91) selon les méthodes standards.
Le séquençage de ces inserts d'ADNc est fait selon la méthode enzymatique de Sanger et coll., Proc. Natl. Acad. Sci. USA. *74*, (1977), p 5463 à l'aide du Sequenase Kit (US Biochemical Corp.)
La séquence nucléotidique d'un clone avec un insert d'ADNc constitué de 462 paires de bases a été établie. Cette séquence est représentée dans la liste des séquences sous le numéro SEQ ID NO: 1 et contient un cadre de lecture ouvert codant pour la pré-xénoxine-1 qui contient 84 acides aminés. Le peptide pré-xénoxine-1 tel que déduit de la séquence d'ADNc commence avec une méthionine d'initiation codée par le codon ATG (pb 39 à 41), suivie d'un peptide signal de 18 acides aminés qui précède la séquence de la xénoxine-1 mature. La séquence d'acides aminés de la xénoxine-1 mature déduite correspond exactement à la séquence complète d'acides aminés déterminée par dégradation d'Edman de la xénoxine-1 purifiée selon l'exemple 1.1 et représentée dans la liste des séquences sous le numéro SEQ ID NO: 3.
Le signal de polyadénylation AATAAA se trouve 12 paires de bases en amont de l'extrémité 3'.

### 2.7. Analyse des séquences peptidiques

Les séquences des xénoxines ont été comparées à la banque de données SWISS-PROT/PIR/GBTRAN release 24 en utilisant PROSCAN, Ver.6.0. du logiciel DNA STAR (DNA STAR Inc).
Cette recherche a seulement permis d'identifier des familles de peptides qui ont une similarité structurelle au niveau du squelette peptidique mais non pas au niveau de l'identité d'acides aminés. Comme illustré à la Figure 3 A, l'identité n'est que de 25,9 % avec un membre représentatif de la famille des cytotoxines, et seulement de 21,3 % avec une neurotoxine courte bien que la configuration des ponts disulfure soit la même. A part les cystéines, ce ne sont que $Asn^{5}$, $Thr^{14}$ et $Asn^{71}$ qui sont conservées dans les trois familles de peptides. Les $Gly^{20}$, $Pro^{50}$ et $Lys^{51}$ qui sont communes aux cytotoxines et aux neurotoxines courtes sont des substitutions non-conservatives comparées aux xénoxines. Les $Arg^{41}$ et $Gly^{42}$ sont délétées chez les xénoxines.
En ce qui concerne le nombre d'acides aminés entre les cystéines on se réfère à la Figure 3B. Pour les trois familles le nombre est comparable du côté N-terminal jusqu'à la $Cys^{3}$ et à partir de la $Cys^{6}$ jusqu'au côté C-terminal. Par contre, chez les xénoxines le nombre de résidus entre les $Cys^{3}$ et $Cys^{4}$ est plus petit et entre les $Cys^{4}$ et $Cys^{5}$ et entre les $Cys^{5}$ et $Cys^{6}$ il est plus important.
Comme illustré dans la Figure 4, la partie C-terminale des xénoxines ($Cys^{37}$ à $Asn^{65}$, 29 résidus) comprenant les $Cys^{4}$ à $Cys^{8}$ partage une identité d'acides aminés de 50 % avec 27 acides aminés ($Cys^{85}$ à $Asn^{111}$) du récepteur humain de l'activine. Du côté N-terminal des xénoxines, on ne retrouve pas d'identité des séquences même pas au niveau des cystéines.

## Exemple 3 : Préparation des xénoxines recombinantes

### 3.1. Construction d'un vecteur d'expression de la xénoxine

Les xénoxines selon l'invention peuvent être obtenues par des techniques de génie génétique, notamment en utilisant comme cellule hôte des levures *Saccharomyces cerevisiae*.
Pour ce faire, on synthétise un fragment d'ADN codant pour la xénoxine-1 mature telle qu'identifiée sous le numéro SEQ ID NO: 1, allant de la base en position 93 jusqu'à la base en position 290 et cloné dans le bactériophage M13TG131 (M.P. Kieny et coll., Gene *26*, (1983), p91).
On construit un plasmide d'expression de la xénoxine-1 qui permet la synthèse et la sécrétion par *S. cerevisiae* selon la demande de brevet internationale PCT/FR90/00306 qui décrit des peptides signal utiles pour la sécrétion d'une protéine hétérologue par la levure.
Le fragment SphI-SmaI de 1045 paires de bases du bactériophage M13TG3846 décrit dans l'exemple 1.E. de ladite demande de brevet internationale est transféré dans le vecteur M13TG3869 (décrit dans l'exemple 1.D. de la même demande) préalablement digéré par SphI et SmaI. On obtient ainsi le vecteur M13TG4803 qui porte :

- le promoteur du gène MF$\alpha$1, suivi d'un codon ATG,
- la séquence XII (voir PCT/FR/90/00306),

- la séquence "pro" mutée du gène MFα1 suivi des codons codant pour Lys-Arg,
- la séquence codant pour rHV2Lys47.

Afin de remplacer la séquence codant pour rHV2Lys47 par celle codant pour la xénoxine-1 on introduit un site HindIII dans la séquence codant pour "pro" mutée de MFα1 selon l'exemple 3.B. de la demande internationale.
Puis, on introduit un fragment HindIII -BamHI qui porte la séquence codant pour la xénoxine-1 dans ce vecteur préalablement traité par HindIII et BamHI pour éliminer la séquence codant pour rHV2Lys47.
Le fragment SphI-SalI de M13TG4803 est introduit dans le plasmide pTG3828 (voir exemple 1.B. de la demande internationale) ouvert aux sites SphI et SalI pour donner le vecteur d'expression de la xénoxine-1 qui comporte :

- la séquence du gène URA3 délétée de son promoteur (URA3-d),
- le promoteur du gène MFα1, suivi d'un ATG,
- la séquence XII comme peptide signal,
- la séquence "pro" mutée du gène MFα1 suivi des codons codant pour Lys-Arg,
- la séquence codant pour la xénoxine-1,
- le terminateur de transcription du gène codant pour PGK de la levure,
- un fragment de pBR322 qui permet la réplication et la sélection chez *E. coli*,
- un fragment du plasmide 2μ qui possède des éléments structuraux nécessaires à la réplication et à l'équipartition mitotique dans la levure.

On transforme une souche de *S. cerevisiae* que l'on mettra en culture dans des conditions connues.

**3.2. Construction de pTG8709 et analyse de la xénoxine-1 mature sécrétée dans le milieu de culture.**

On amplifie par PCR la séquence codant pour la xénoxine-1 mature munie d'un site EagI en 5' et d'un site Sali en 3'. On utilise à titre de matrice M13TG4414 ou pTG4414 (Kolbe et coll., J. Biol. Chem., *268,* (1993) p. 16458) et les amorces :

OTG5770 :
5'-TACGTCGGCCGTTACAAGAGACTGAAATGTGTGAATTTACAAGCG-3'

et

OTG5771 :
5'-AGTTTGTCGACTCAAGCATTGCACATGTTTCCT-3'

Le fragment ainsi généré est intégré dans M13TG9800 préalablement digéré par EagI et Sali pour donner M13TG8703. Le vecteur M13TG9800 est issu de M13TG131 dans lequel sont insérés les éléments essentiels à l'expression d'une protéine d'intérêt, à savoir :

- Le promoteur du gène MFα1 suivi d'un codon ATG (Inokuchi et coll., Mol. Cell. Biol. *7* (1987) p. 3185)
- La séquence pré BGL2 (β-glucanase 2 ; Klebl et Tanner, J. Bacteriol., *171* (1989) p. 6259
- La séquence pro de la défensine A (Dimarcq et coll., EMBO J. 9 (1990) p. 2507) dans laquelle on a introduit, de manière silencieuse, un site EagI à son extrémité 3' (couvrant les codons précédents ceux codant pour les résidus Lys-Arg en 3').
- Des sites de restriction pour l'insertion d'une séquence d'ADN d'intérêt.

On introduit le fragment SphI-SalI isolé de M13TG8703 et portant la cassette d'expression de la xénoxine-1 mature entre les mêmes sites de pTG4812 pour obtenir pTG8709. Le vecteur pTG4812 est dérivé de pTG3828 mais comprend en outre une cassette pour l'expression du gène KEX2 de levure insérée au niveau de la jonction de la partie 2μ et de la partie pBR322.
pTG8709 est transformé dans une souche *Saccharomyces cerevisiae.* On peut citer à titre d'exemples TGY47.1 (MATα, pral, prb1, prc1, cps1, ura3-delta5, leu2-3, leu 2-112, his) ou TGY73.4 qui dérive de la précédente mais présente une prototrophie pour la leucine (Leu+).

On sélectionne un transformant TGY73.4/pTG8709 que l'on cultive à 28°C en milieu sélectif minimum (0,67% de bases azotées pour levure YNB (Yeast Nitrogen Base) sans acide aminé, 1% de casaminoacides, 2% de glucose et 2% de bactoagar).

Après 60 heures de culture, le surnageant de culture est concentré par ultrafiltration sur membrane YM2/centrifugation selon les techniques conventionnelles. Le concentré est soumis à une éléctrophorèse dénaturante sur gel de polyacrylamide (15,3 % en polyacrylamide). Les protéines sont transférées sur membrane PVDF et le matériel correspond au poids moléculaire attendu (environ 7kDa) est soumis à un séquençage des acides aminés N-terminaux.

On lit, de façon majoritaire, une séquence qui correspond à la séquence N-terminale attendue pour la xénoxine-1 mature.

**Revendications**

1. Peptide caractérisé en ce qu'il comprend une séquence d'acides aminés, laquelle :

   a. contient au moins 8 cystéines qui sont reliées par 4 ponts disulfure suivant la configuration $Cys^1$ avec $Cys^3$, $Cys^2$ avec $Cys^4$, $Cys^5$ avec $Cys^6$ et $Cys^7$ avec $Cys^8$ ;

   b. contient 0 à 3 acides aminés du côté N-terminal de la $Cys^1$, 9 à 14 acides aminés entre les $Cys^1$ et $Cys^2$, 3 à 7 acides aminés entre les $Cys^2$ et $Cys^3$, II à 18 acides aminés entre les $Cys^3$ et $Cys^4$, 1 à 6 acides aminés entre les $Cys^4$ et $Cys^5$, 7 à 15 acides aminés entre les $Cys^5$ et $Cys^6$, aucun acide aminé entre les $Cys^6$ et $Cys^7$, 3 à 5 acides aminés entre les $Cys^7$ et $Cys^8$ et 0 à 10 acides aminés du côté C-terminal de la $Cys^8$; et

   c. présente une identité d'acides aminés après alignement, d'au moins 40 % avec la séquence d'acides aminés identifiée sous le numéro SEQ ID NO: 3.

2. Peptide caractérisé en ce qu'il comprend une séquence d'acides aminés, laquelle :

   a. contient au moins 8 cystéines qui , lorsque le peptide adopte sa conformation repliée, sont reliées par 4 ponts disulfure suivant la configuration $Cys^1$ avec $Cys^3$, $Cys^2$ avec $Cys^4$, $Cys^5$ avec $Cys^6$ et $Cys^7$ avec $Cys^8$ ;

   b. contient 0 à 3 acides aminés du côté N-terminal de la $Cys^1$, 9 à 14 acides aminés entre les $Cys^1$ et $Cys^2$, 3 à 7 acides aminés entre les $Cys^2$ et $Cys^3$, 11 à 18 acides aminés entre les $Cys^3$ et $Cys^4$, 1 à 6 acides aminés entre les $Cys^4$ et $Cys^5$, 7 à 15 acides aminés entre les $Cys^5$ et $Cys^6$, aucun acide aminé entre les $Cys^6$ et $Cys^7$, 3 à 5 acides aminés entre les $Cys^7$ et $Cys^8$ et 0 à 10 acides aminés du côté C-terminal de la $Cys^8$ ; et

   c. présente une identité d'acides aminés après alignement, d'au moins 40% avec la séquence d'acides aminés identifiée sous le numéro SEQ ID NO: 3.

3. Un peptide selon la revendication 1 ou 2, caractérisé en ce que la séquence d'acides aminés contient 2 acides aminés du côté N-terminal de la $Cys^1$, 10 à 13 acides aminés entre les $Cys^1$ et $Cys^2$, 4 à 6 acides aminés entre les $Cys^2$ et $Cys^3$, 12 à 17 acides aminés entre les $Cys^3$ et $Cys^4$, 1 à 5 acides aminés entre $Cys^4$ et $Cys^5$, 8 à 14 acides aminés entre les $Cys^5$ et $Cys^6$, aucun acide aminé entre les $Cys^6$ et $Cys^7$, 4 acides aminés entre les $Cys^7$ et $Cys^8$ et 2 acides aminés du côté C-terminal de la $Cys^8$.

4. Un peptide selon l'une des revendications 1 à 3, caractérise en ce que la séquence d'acides aminés présente une identité d'acides aminés d'au moins 50%, de préférence au moins 60%, avec la séquence d'acides aminés identifiée sous le numéro SEQ ID NO : 3.

**5.** Un peptide selon la revendication 4, caractérisé en ce que l'identité d'acides aminés soit au moins 70%.

**6.** Un peptide selon la revendication 1 ou 2, caractérisé en ce qu'il comprend une séquence d'acides aminés laquelle contient 0 à 3 acides aminés du côté N-terminal de la $Cys^1$, 13 acides aminés entre les $Cys^1$ et $Cys^2$, 6 acides aminés entre les $Cys^2$ et $Cys^3$, 12 acides aminés entre les $Cys^3$ et $Cys^4$, 5 acides aminés entre les $Cys^4$ et $Cys^5$, 14 acides aminés entre les $Cys^5$ et $Cys^6$, aucun acide aminé entre les $Cys^6$ et $Cys^7$, 4 acides aminés entre les $Cys^7$ et $Cys^8$ et 0 à 10 acides aminés du côté C-terminal de la $Cys^8$, et qui présente une identité d'acides aminés après alignement, d'au moins 80%, de préférence 90%, avec la séquence d'acides aminés identifiées sous le numéro SEQ ID NO : 3.

**7.** Un peptide selon la revendication 6 caractérisé en ce que l'identité d'acides aminés est au moins une séquence telle que identifiée sous le numéro SEQ ID NO: 4 ou SEQ ID NO: 5.

**8.** Un peptide selon la revendication 6, caractérisé en ce que l'identité d'acides aminés est au moins 95% avec la séquence d'acides aminés identifiée sous le numéro SEQ ID NO: 3.

**9.** Un peptide selon la revendication 8, caractérisé en ce que la séquence d'acides aminés comprend une séquence telle que identifiée sous le numéro SEQ ID NO: 3.

**10.** Un procédé de préparation d'un peptide selon l'une des revendications 1 à 9, caractérisé en ce que pour isoler ledit peptide à partir de la matière sécrétée par la peau d'une grenouille, de préférence de *Xenopus laevis,*

a. on divise en fractions la matière sécrétée par la peau de grenouille par précipitation à l'aide de sulfate d'ammonium, ou d'un agent précipitant équivalent,

b. on soumet les précipités qui se forment entre 30 % et 70 % de saturation en sulfate d'ammonium, de préférence entre 30 % et 60 %, ou en conditions équivalentes, à une chromatographie par perméation de gel, et

c. on récolte la fraction peptidique qui élue tardivement,
et si nécessaire,

d. on soumet cette fraction peptidique à une chromatographie HPLC d'échange de cations éventuellement suivi d'une chromatographie HPLC phase inverse.

**11.** Un fragment d'ADN isolé caractérisé en ce qu'il comprend une séquence codant pour un peptide selon l'une des revendications 1 à 9.

**12.** Un fragment d'ADN selon la revendication 11, caractérisé en ce qu'il comprend une séquence codant pour le précurseur d'un peptide selon l'une des revendications 1 à 9.

**13.** Un fragment d'ADN selon la revendication 11, caractérisé en ce que la séquence code pour une séquence d'acides aminés telle que identifiée sous le numéro SEQ ID NO: 3, SEQ ID NO: 4 ou SEQ ID NO: 5.

**14.** Un fragment d'ADN selon la revendication 11 caractérisé en ce qu'il comprend une séquence d'acides nucléiques codant pour une séquence d'acides aminés telle que identifiée sous le numéro SEQ ID NO: 1 et allant de la base en position 93 jusqu'à la base en position 290.

**15.** Un fragment d'ADN selon la revendication 12 caractérisé en ce qu'il comprend une séquence d'acides nucléiques codant pour une séquence d'acides aminés telle que identifiée sous le numéro SEQ ID NO: 1 et allant de l'acide nucléique en position 39 jusqu'à l'acide nucléique en position 290.

**16.** Une cassette d'expression d'un fragment d'ADN isolé selon l'une des revendications 11 à 15, caractérisée en ce que ledit fragment d'ADN est placé sous le contrôle d'éléments permettant sa transcription et/ou sa traduction dans la cellule transformée considérée.

**17.** Une cassette d'expression selon la revendication 16, caractérisée en ce qu'elle comprend en outre une séquence pré et une séquence pro, notamment la séquence pro de la défensine A de *Phormia terranovae*.

**18.** Une cassette d'expression selon la revendication 16 ou 17, caractérisée en ce qu'elle comprend un fragment d'ADN selon la revendication 12.

**19.** Une cellule transformée avec une cassette d'expression selon l'une des revendications 16 à 18, caractérisée en ce que ladite cassette d'expression est soit intégrée dans le génome de la cellule transformée ou soit portée par un vecteur d'expression.

**20.** Une cellule transformée selon la revendication 19, caractérisée en ce qu'elle est choisie parmi le groupe constitué de *S. cerevisiae* et *E. coli*.

**21.** Une cellule transformée selon la revendication 19, caractérisée en ce que la cellule est d'origine mammifère.

**22.** Procédé de préparation d'un peptide selon l'une des revendications 1 à 9, caractérisé en ce qu'on cultive une cellule transformée selon l'une des revendications 19 à 21 sur un milieu approprié et l'on récupère le peptide à partir de ladite culture.

**23.** Composition pharmaceutique à usage humain ou vétérinaire caractérisée en ce qu'elle comprend comme agent actif au moins un peptide selon l'une des revendications 1 à 9, un vecteur contenant une cassette d'expression selon l'une des revendications 16 à 18 ou une cellule transformée selon l'une des revendications 19 à 21.

**24.** Un anticorps, monoclonal ou polyclonal, caractérisé en ce qu'il reconnaît un peptide selon l'une des revendications 1 à 9.

**25.** Trousse de diagnostic comprenant un peptide selon l'une des revendications 1 à 9 ou un anticorps selon la revendication 24.

## Claims

**1.** Peptide, characterized in that it comprises an amino acid sequence which:

a. contains at least 8 cysteines which are linked via 4 disulphide bridges according to the configuration $Cys^1$ with $Cys^3$, $Cys^2$ with $Cys^4$, $Cys^5$ with $Cys^6$ and $Cys^7$ with $Cys^8$;
b. contains 0 to 3 amino acids on the N-terminal side of $Cys^1$, 9 to 14 amino acids between $Cys^1$ and $Cys^2$, 3 to 7 amino acids between $Cys^2$ and $Cys^3$, 11 to 18 amino acids between $Cys^3$ and $Cys^4$, 1 to 6 amino acids between $Cys^4$ and $Cys^5$, 7 to 15 amino acids between $Cys^5$ and $Cys^6$, no amino acid between $Cys^6$ and $Cys^7$, 3 to 5 amino acids between $Cys^7$ and $Cys^8$ and 0 to 10 amino acids on the C-terminal side of $Cys^8$; and
c. displays at least 40% amino acid identity, after alignment, with the amino acid sequence identified under the number SEQ ID NO: 3.

**2.** Peptide, characterized in that it comprises an amino acid sequence which:

a. contains at least 8 cysteines which, when the peptide adopts its folded conformation, are linked via 4 disulphide bridges according to the configuration $Cys^1$ with $Cys^3$, $Cys^2$ with $Cys^4$, $Cys^5$ with $Cys^6$ and $Cys^7$ with $Cys^8$;
b. contains 0 to 3 amino acids on the N-terminal side of $Cys^1$, 9 to 14 amino acids between $Cys^1$ and $Cys^2$, 3 to 7 amino acids between $Cys^2$ and $Cys^3$, 11 to 18 amino acids between $Cys^3$ and $Cys^4$, 1 to 6 amino acids between $Cys^4$ and $Cys^5$, 7 to 15 amino acids between $Cys^5$ and $Cys^6$, no amino acid between $Cys^6$ and $Cys^7$, 3 to 5 amino acids between $Cys^7$ and $Cys^8$ and 0 to 10 amino acids on the C-terminal side of $Cys^8$; and
c. displays at least 40% amino acid identity, after alignment, with the amino acid sequence identified under the number SEQ ID NO: 3.

**3.** A peptide according to Claim 1 or 2, characterized in that the amino acid sequence contains 2 amino acids on the N-terminal side of $Cys^1$, 10 to 13 amino acids between $Cys^1$ and $Cys^2$, 4 to 6 amino acid between $Cys^2$ and $Cys^3$, 12 to 17 amino acids between $Cys^3$ and $Cys^4$, 1 to 5 amino acids between $Cys^4$ and $Cys^5$, 8 to 14 amino acids

between $Cys^5$ and $Cys^6$, no amino acids between $Cys^6$ and $Cys^7$, 4 amino acids between $Cys^7$ and $Cys^8$ and 2 amino acids on the C-terminal side of $Cys^8$.

**4.** A peptide according to one of Claims 1 to 3, characterized in that the amino acid sequence displays at least 50%, and preferably at least 60%, amino acid identity with the amino acid sequence identified under the number SEQ ID NO: 3.

**5.** A peptide according to Claim 4, characterized in that the amino acid identity is at least 70%.

**6.** A peptide according to Claim 1 or 2, characterized in that it comprises an amino acid sequence which contains 0 to 3 amino acids on the N-terminal side of $Cys^1$, 13 amino acids between $Cys^1$ and $Cys^2$, 6 amino acids between $Cys^2$ and $Cys^3$, 12 amino acids between $Cys^3$ and $Cys^4$, 5 amino acids between $Cys^4$ and $Cys^5$, 14 amino acids between $Cys^5$ and $Cys^6$, no amino acid between $Cys^6$ and $Cys^7$, 4 amino acids between $Cys^7$ and $Cys^8$ and 0 to 10 amino acids on the C-terminal side of $Cys^8$, and which displays at least 80%, and preferably 90%, amino acid identity, after alignment, with the amino acid sequence identified under the number SEQ ID NO: 3.

**7.** A peptide according to Claim 6, characterized in that the amino acid identity is at least one sequence as defined under the number SEQ ID NO: 4 or SEQ ID NO: 5.

**8.** A peptide according to Claim 6, characterized in that the amino acid identity with the amino acid sequence identified under the number SEQ ID NO: 3 is at least 95%.

**9.** A peptide according to Claim 8, characterized in that the amino acid sequence comprises a sequence as identified under the number SEQ ID NO: 3.

**10.** A method for preparing a peptide according to one of Claims 1 to 9, characterized in that, in order to isolate said peptide from the matter secreted by the skin of a frog, preferably *Xenopus laevis,*

a. the matter secreted by the skin of a frog is divided into fractions by precipitation using ammonium sulphate or an equivalent precipitating agent,
b. the precipitates which form at between 30% and 70% saturation with ammonium sulphate, and preferably between 30% and 60%, or under equivalent conditions, are subjected to gel permeation chromatography, and
c. the peptide fraction which elutes late on is recovered,
and, if necessary,
d. this peptide fraction is subjected to cation exchange HPLC chromatography optionally followed by reverse-phase HPLC chromatography.

**11.** An isolated DNA fragment, characterized in that it comprises a sequence coding for a peptide according to Claims 1 to 9.

**12.** A DNA fragment according to Claim 11, characterized in that it comprises a sequence coding for the precursor of a peptide according to one of Claims 1 to 9.

**13.** A DNA fragment according to Claim 11, characterized in that the sequence codes for an amino acid sequence as identified under the number SEQ ID NO: 3, SEQ ID NO: 4 or SEQ ID NO: 5.

**14.** A DNA fragment according to Claim 11, characterized in that it comprises a nucleic acid sequence coding for an amino acid sequence as identified under the number SEQ ID NO: 1 and extending from the base at position 93 to the base at position 290.

**15.** A DNA fragment according to Claim 12, characterized in that it comprises a nucleic acid sequence coding for an amino acid sequence as identified under the number SEQ ID NO: 1 and extending from the nucleic acid at position 39 to the nucleic acid at position 290.

**16.** A cassette for the expression of an isolated DNA fragment according to one of Claims 11 to 15, characterized in that said DNA fragment is placed under the control of elements permitting its transcription and/or its translation in the transformed cell in question.

**17.** An expression cassette according to Claim 16, characterized in that it comprises, in addition, a pre sequence and a pro sequence, in particular the pro sequence of *Phormia terranovae* defensin A.

**18.** An expression cassette according to Claim 16 or 17, characterized in that it comprises a DNA fragment according to Claim 12.

**19.** A cell transformed with an expression cassette according to one of Claims 16 to 18, characterized in that said expression cassette is either integrated in the genome of the transformed cell or is carried by an expression vector.

**20.** A transformed cell according to Claim 19, characterized in that it is chosen from the group consisting of *S. cerevisiae* and *E. coli*.

**21.** A transformed cell according to Claim 19, characterized in that the cell is of mammalian origin.

**22.** Method for preparing a peptide according to one of Claims 1 to 9, characterized in that a transformed cell according to one of Claims 19 to 21 is cultured on a suitable medium and the peptide is recovered from said culture.

**23.** Pharmaceutical composition for human or veterinary use, characterized in that it comprises as active agent at least one peptide according to one of Claims 1 to 9, one vector containing an expression cassette according to one of Claims 16 to 18 or one transformed cell according to one of Claims 19 to 21.

**24.** A monoclonal or polyclonal antibody, characterized in that it recognizes a peptide according to one of Claims 1 to 9.

**25.** Diagnostic kit comprising a peptide according to one of Claims 1 to 9 or an antibody according to Claim 24.

**Patentansprüche**

**1.** Peptid, dadurch gekennzeichnet, dass es eine Aminosäuresequenz aufweist, welche:

a. wenigstens 8 Cysteine enthält, die durch 4 Disulfidbrücken gemäß der Konfiguration $Cys^1$ mit $Cys^3$, $Cys^2$ mit $Cys^4$, $Cys^5$ mit $Cys^6$ und $Cys^7$ mit $Cys^8$ miteinander verbunden sind;

b. 0 bis 3 Aminosäuren auf der N-terminalen Seite des $Cys^1$, 9 bis 14 Aminosäuren zwischen den $Cys^1$ und $Cys^2$, 3 bis 7 Aminosäuren zwischen den $Cys^2$ und $Cys^3$, 11 bis 18 Aminosäuren zwischen den $Cys^3$ und $Cys^4$, 1 bis 6 Aminosäuren zwischen den $Cys^4$ und $Cys^5$, 7 bis 15 Aminosäuren zwischen den $Cys^5$ und $Cys^6$, keine Aminosäure zwischen den $Cys^6$ und $Cys^7$, 3 bis 5 Aminosäuren zwischen den $Cys^7$ und $Cys^8$ und 0 bis 10 Aminosäuren auf der C-terminalen Seite des $Cys^8$ enthält; und

c. eine Identität der Aminosäuren in ihrer Anordnung von wenigstens 40 % mit der unter der Nummer SEQ ID NO: 3 aufgeführten Aminosäuresequenz aufweist.

**2.** Peptid, dadurch gekennzeichnet, dass es eine Aminosäuresequenz aufweist, welche:

a. wenigstens 8 Cysteine enthält, die, wenn das Peptid seine zusammengefaltete Struktur annimmt, durch 4 Disulfidbrücken gemäß der Konfiguration $Cys^1$ mit $Cys^3$, $Cys^2$ mit $Cys^4$, $Cys^5$ mit $Cys^6$ und $Cys^7$ mit $Cys^8$ miteinander verbunden sind;

b. 0 bis 3 Aminosäuren auf der N-terminalen Seite des $Cys^1$, 9 bis 14 Aminosäuren zwischen den $Cys^1$ und $Cys^2$, 3 bis 7 Aminosäuren zwischen den $Cys^2$ und $Cys^3$, 11 bis 18 Aminosäuren zwischen den $Cys^3$ und $Cys^4$, 1 bis 6 Aminosäuren zwischen den $Cys^4$ und $Cys^5$, 7 bis 15 Aminosäuren zwischen den $Cys^5$ und $Cys^6$, keine Aminosäure zwischen den $Cys^6$ und $Cys^7$, 3 bis 5 Aminosäuren zwischen den $Cys^7$ und $Cys^8$ und 0 bis 10 Aminosäuren auf der C-terminalen Seite des $Cys^8$ enthält; und

c. eine Identität der Aminosäuren in ihrer Anordnung von wenigstens 40 % mit der unter der Nummer SEQ ID NO: 3 aufgeführten Aminosäuresequenz aufweist.

**3.** Peptid gemäß Anspruch 1 oder 2, dadurch gekennzeichnet, dass die Aminosäuresequenz 2 Aminosäuren auf der N-terminalen Seite des $Cys^1$, 10 bis 13 Aminosäuren zwischen den $Cys^1$ und $Cys^2$, 4 bis 6 Aminosäuren zwischen den $Cys^2$ und $Cys^3$, 12 bis 17 Aminosäuren zwischen den $Cys^3$ und $Cys^4$, 1 bis 5 Aminosäuren zwischen den $Cys^4$ und $Cys^5$, 8 bis 14 Aminosäuren zwischen den $Cys^5$ und $Cys^6$, keine Aminosäure zwischen den $Cys^6$ und $Cys^7$, 4 Aminosäuren zwischen den $Cys^7$ und $Cys^8$ und 2 Aminosäuren auf der C-terminalen Seite des $Cys^8$ enthält.

**4.** Peptid gemäß einem der Ansprüche 1 bis 3, dadurch gekennzeichnet, dass die Aminosäuresequenz eine Aminosäureidentität von wenigstens 50 %, bevorzugt wenigstens 60 %, mit der unter der Nummer SEQ ID NO: 3 aufgeführten Aminosäuresequenz aufweist.

**5.** Peptid gemäß Anspruch 4, dadurch gekennzeichnet, dass die Aminosäureidentität wenigstens 70 % beträgt.

**6.** Peptid gemäß Anspruch 1 oder 2, dadurch gekennzeichnet, dass es eine Aminosäuresequenz aufweist, die 0 bis 3 Aminosäuren auf der N-terminalen Seite des $Cys^1$, 13 Aminosäuren zwischen den $Cys^1$ und $Cys^2$, 6 Aminosäuren zwischen den $Cys^2$ und $Cys^3$, 12 Aminosäuren zwischen den $Cys^3$ und $Cys^4$, 5 Aminosäuren zwischen den $Cys^4$ und $Cys^5$, 14 Aminosäuren zwischen den $Cys^5$ und $Cys^6$, keine Aminosäure zwischen $Cys^6$ und $Cys^7$, 4 Aminosäuren zwischen den $Cys^7$ und $Cys^8$ und 0 bis 10 Aminosäuren auf der C-terminalen Seite des $Cys^8$ enthält, und eine Identität der Aminosäuren in ihrer Anordnung von wenigstens 80 %, bevorzugt 90 %, mit der unter der Nummer SEQ ID NO: 3 aufgeführten Aminosäuresequenz aufweist.

**7.** Peptid gemäß Anspruch 6, dadurch gekennzeichnet, dass sich die Aminosäureidentität auf wenigstens eine der unter den Nummern SEQ ID NO: 4 oder SEQ ID NO: 5 aufgeführten Sequenzen bezieht.

**8.** Peptid gemäß Anspruch 6, dadurch gekennzeichnet, dass die Aminosäureidentität wenigstens 95 % mit der unter der Nummer SEQ ID NO: 3 aufgeführten Aminosäuresequenz beträgt.

**9.** Peptid gemäß Anspruch 8, dadurch gekennzeichnet, dass die Aminosäuresequenz die unter der Nummer SEQ ID NO: 3 aufgeführte Sequenz umfasst.

**10.** Verfahren zur Herstellung eines Peptids gemäß einem der Ansprüche 1 bis 9, dadurch gekennzeichnet, dass man zur Isolierung des Peptids, ausgehend von durch die Haut eines Frosches, bevorzugt *Xenopus laevis,* sekretiertem Material,

a. das durch die Haut des Frosches sekretierte Material durch Ausfälllung mit Hilfe von Ammoniumsulfat oder einem gleichwertigen Ausfällungsmittel in Fraktionen teilt,

b. die sich zwischen 30- und 70%iger, bevorzugt zwischen 30- und 60%iger Ammoniumsulfatsättigung oder unter gleichwertigen Bedingungen bildenden Präzipitate einer Gelpermeationschromatographie unterwirft und

c. die spät eluierte peptidische Fraktion gewinnt,
und, wenn notwendig,

d. diese Peptidfraktion einer HPLC-Ionenaustauschchromatographie und gegebenenfalls einer anschließenden HPLC-Umkehrphasenchromatographie unterwirft.

**11.** Isoliertes DNA-Fragment, dadurch gekennzeichnet, dass es eine Sequenz aufweist, welche für ein Peptid gemäß einem der Ansprüche 1 bis 9 kodiert.

**12.** DNA-Fragment gemäß Anspruch 11, dadurch gekennzeichnet, dass es eine Sequenz aufweist, welche für den Vorläufer eines Peptids gemäß einem der Ansprüche 1 bis 9 kodiert.

**13.** DNA-Fragment gemäß Anspruch 11, dadurch gekennzeichnet, dass die Sequenz für eine der unter den Nummern SEQ ID NO: 3, SEQ ID NO: 4 oder SEQ ID NO: 5 aufgeführten Aminosäuresequenzen kodiert.

**14.** DNA-Fragment gemäß Anspruch 11, dadurch gekennzeichnet, dass es eine Nukleinsäuresequenz aufweist, welche für die unter der Nummer SEQ ID NO: 1 aufgeführte Aminosäuresequenz kodiert und sich von der Base an Position 93 bis zur Base an Position 290 erstreckt.

**15.** DNA-Fragment gemäß Anspruch 12, dadurch gekennzeichnet, dass es eine Nukleinsäuresequenz aufweist, welche für die unter der Nummer SEQ ID NO: 1 aufgeführte Aminosäuresequenz kodiert und sich von der Nukleinsäure an Position 39 bis zur Nukleinsäure an Position 290 erstreckt.

**16.** Expressionskassette eines isolierten DNA-Fragments gemäß einem der Ansprüche 11 bis 15, dadurch gekennzeichnet, dass das DNA-Fragment unter der Kontrolle von Elementen platziert ist, die seine Transkription und/oder Translation in der betreffenden transformierten Zelle ermöglichen.

**17.** Expressionskassette gemäß Anspruch 16, dadurch gekennzeichnet, dass sie außerdem eine Prä- und eine Pro-Sequenz, insbesondere die Pro-Sequenz des Defensin A von *Phormia terranovae*, enthält.

**18.** Expressionskassette gemäß Anspruch 16 oder 17, dadurch gekennzeichnet, dass sie ein DNA-Fragment gemäß Anspruch 12 enthält.

**19.** Zelle, welche mit einer Expressionskassette gemäß einem der Ansprüche 16 bis 18 transformiert ist, dadurch gekennzeichnet, dass die Expressionskassette entweder in das Genom der transformierten Zelle integriert ist oder von einem Expressionsvektor getragen wird.

**20.** Transformierte Zelle gemäß Anspruch 19, dadurch gekennzeichnet, dass es sich um eine *S. cerevisiae*- oder *E. coli*-Zelle handelt.

**21.** Transformierte Zelle gemäß Anspruch 19, dadurch gekennzeichnet, dass es sich um eine Säugetierzelle handelt.

**22.** Verfahren zur Herstellung eines Peptids gemäß einem der Ansprüche 1 bis 9, dadurch gekennzeichnet, dass man eine transformierte Zelle gemäß einem der Ansprüche 19 bis 21 in einem geeigneten Medium kultiviert und das Peptid aus der Kultur gewinnt.

**23.** Pharmazeutische Zusammensetzung zur Verwendung in der Human- oder Tiermedizin, dadurch gekennzeichnet, dass sie als Wirkstoff wenigstens ein Peptid gemäß einem der Ansprüche 1 bis 9, einen Vektor, enthaltend eine Expressionskassette gemäß einem der Ansprüche 16 bis 18, oder eine transformierte Zelle gemäß einem der Ansprüche 19 bis 21 enthält.

**24.** Monoklonaler oder polyklonaler Antikörper, dadurch gekennzeichnet, dass er ein Peptid gemäß einem der Ansprüche 1 bis 9 erkennt.

**25.** Diagnostik-System, welches ein Peptid gemäß einem der Ansprüche 1 bis 9 oder einen Antikörper gemäß Anspruch 24 enthält.

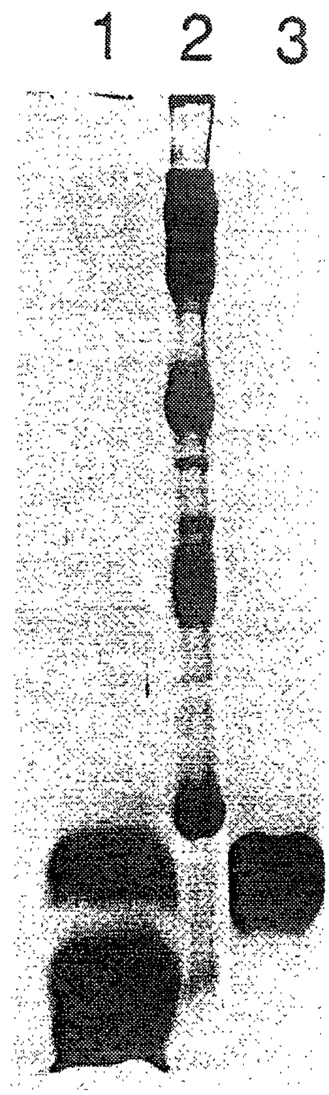


FIGURE 1

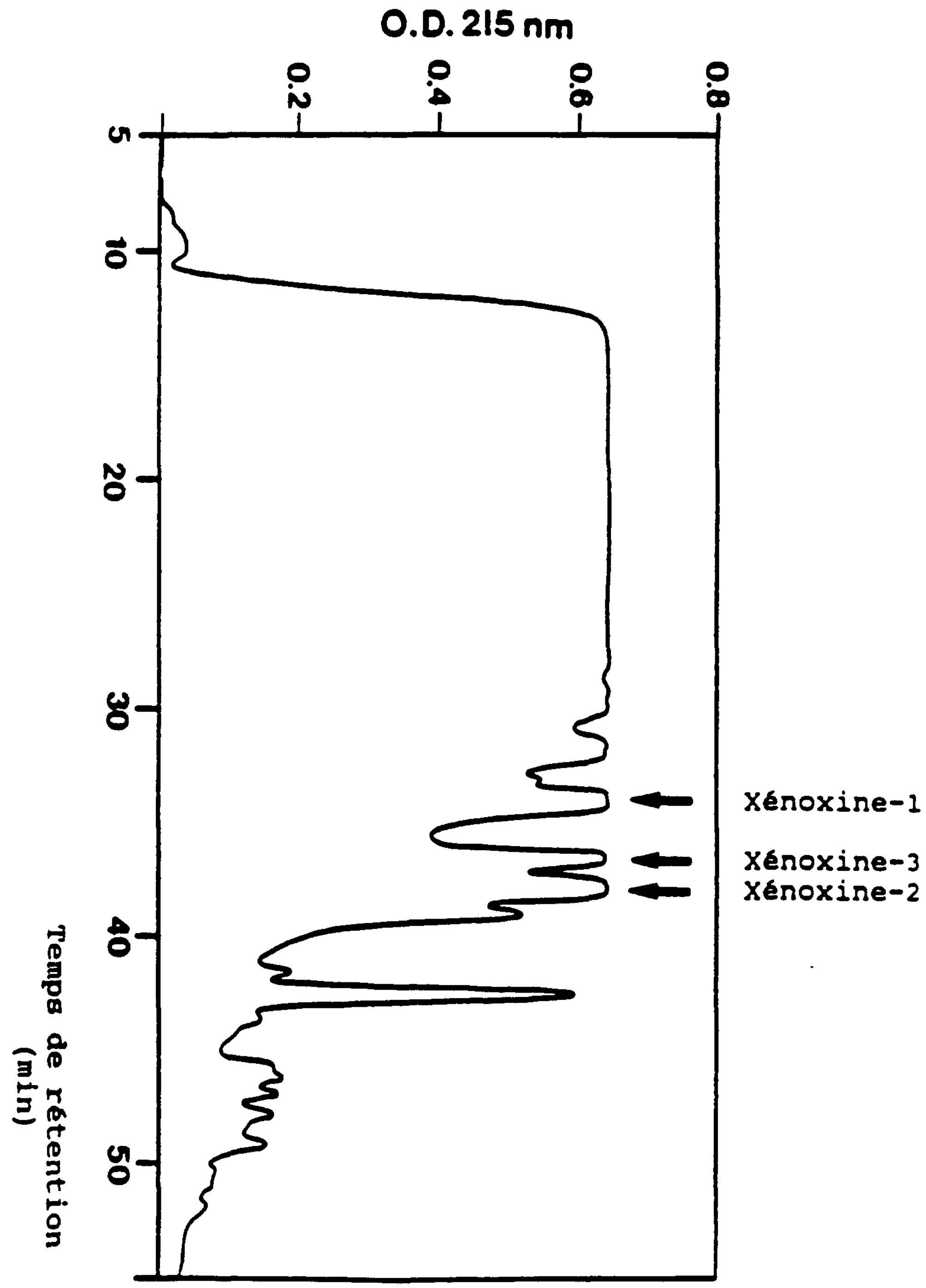
O.D. 215 nm
0.2
0.4
0.6
0.8
5
10
20
30
40
50
Temps de rétention
(min)
Xénoxine-1
Xénoxine-3
Xénoxine-2

FIGURE 2

**FIGURE 3A**

| | |
|---|---|
| Xénoxine[a] | LKCVNLQANGIKMTQECAKEDTKCLTLRSLKKT-LKF-----CASGRTCTTMKIMSLPGEQITCCEGNMCNA |
| Cytotoxine[b] | LKCHNTQLPFIYKT--CPEGKNLCFKA-TLKKFPLKIPIKRGCAD--NCPKNSALL----KYVCCSTDKCN |
| Neurotoxine[c] | RRCFNQQSSQPKTTKSCPPGENSCYNKQWRDHRGSI--TERGCG----CPKVK----PGIKLRCCESEDCNN |

**FIGURE 3B**

| | | C | | C | | C | | C | | C | | CC | | C | |
|---|---|---|---|---|---|---|---|---|---|---|---|---|---|---|---|
| XENOXINES | 2 | | 13 | | 6 | | 12 | | 5 | | 14 | | 4 | | 2 |
| CYTOTOXINES | 2 | | 10-11 | | 6 | | 15-17 | | 3 | | 10 | | 4 | | 1-2 |
| NEUROTOXINES | 2 | | 12-13 | | 4-6 | | 15-16 | | 1 | | 8-11 | | 4 | | 2 |

```
                        |                |      |              |      |                ||   |
Xénoxine-1              LKCVNLQANGIKMTQECAKEDTKCLTLRSLKKTLKFCASGRT-CTTMKIMSLPGEQITCCEGNMCNA
                                                            C    RT C   K  S P     CCEGNMCN

Récepteur             VEPCYGDKDKRRHCFATWKNISGSIEIVKQGCWLDDINCY-DRTDCVEKK-DS-PEVYFCCCEGNMCNEKF
humain d'activine       |          |                  |      |      |              |||   |
```

FIGURE 4

LISTE DE SEQUENCES

(1) INFORMATION GENERALE:

(i) DEPOSANT:

(A) NOM: TRANSGENE S.A.
(B) RUE: 11, RUE DE MOLSHEIM
(C) VILLE: STRASBOURG CEDEX
(E) PAYS: FRANCE
(F) CODE POSTAL: 67082
(G) TELEPHONE: (33) 88 27 91 00
(H) TELECOPIE: (33) 88 22 58 07

(ii) TITRE DE L'INVENTION: Famille de peptides, appelés xénoxines.

(iii) NOMBRE DE SEQUENCES: 28

(iv) FORME LISIBLE PAR ORDINATEUR:

(A) TYPE DE SUPPORT: Floppy disk
(B) ORDINATEUR: IBM PC compatible
(C) SYSTEME D' EXPLOITATION: PC-DOS/MS-DOS
(D) LOGICIEL: Patentin Release #1.0, Version #1.25 (OEB)

(2)INFORMATION POUR LA SEQ ID NO: 1:

(i) CARACTERISTIQUES DE LA SEQUENCE:

(A) LONGUEUR: 462 paires de bases
(B) TYPE: acide nucléique
(C) NOMBRE DE BRINS: simple
(D) CONFIGURATION: linéaire

(ii) TYPE DE MOLECULE: ADNc pour ARNm

(ix) CARACTERISTIQUE ADDITIONELLE:

(A) NOM/CLE: CDS
(B) EMPLACEMENT: 39..293
(D) AUTRES RENSEIGNEMENTS: /codon_start= 39
/product= "prexenoxine-1"
/note= "le peptide signal comprend les dix-huit premier residus d'acides amines"

(xi) DESCRIPTION DE LA SEQUENCE: SEQ ID NO: 1:

```
AAGATGTGCT GTGATTGGTT GAAAGCTTGA GCCACACA ATG CGT TAC GCC ATC        53
                                          Met Arg Tyr Ala Ile
                                           1               5

GTC TTC TTT CTC GTT TGT GTC ATT ACT CTT GGA GAA GCA CTG AAA TGT     101
Val Phe Phe Leu Val Cys Val Ile Thr Leu Gly Glu Ala Leu Lys Cys
                 10                  15                  20

GTG AAT TTA CAA GCG AAT GGA ATA AAG ATG ACA CAA GAG TGT GCA AAG     149
Val Asn Leu Gln Ala Asn Gly Ile Lys Met Thr Gln Glu Cys Ala Lys
             25                  30                  35

GAG GAT ACC AAA TGC TTA ACA TTA AGA TCA TTA AAA AAA ACT TTA AAG     197
Glu Asp Thr Lys Cys Leu Thr Leu Arg Ser Leu Lys Lys Thr Leu Lys
         40                  45                  50

TTT TGT GCC TCT GGT CGA ACA TGT ACG ACT ATG AAA ATA ATG TCT TTG     245
Phe Cys Ala Ser Gly Arg Thr Cys Thr Thr Met Lys Ile Met Ser Leu
     55                  60                  65

CCT GGG GAA CAG ATT ACA TGC TGT GAA GGA AAC ATG TGC AAT GCT TGAGATGATT   300
Pro Gly Glu Gln Ile Thr Cys Cys Glu Gly Asn Met Cys Asn Ala
 70                  75                  80                  85

CTTCCAATGG AGGTTGCCGG GATTTTCTCT TCTCACATGA AGCAATGGCC CTACCAAAAC    360

ATTTGTCCTC TCCTCTCTCC TCTTTTCCCT TCTGTCCACT CTCCTATAGA TACTAGGGTG    420

TAGCTTGATT CCTAATATCC ATTAAATAAA GCACTCAACT GC                      462
```

(2) INFORMATION POUR LA SEQ ID NO: 2:

(i) CARACTERISTIQUES DE LA SEQUENCE:

(A) LONGUEUR: 84 acides aminés
(B) TYPE: acide aminé
(D) CONFIGURATION: linéaire

(ii) TYPE DE MOLECULE: peptide

(xi) DESCRIPTION DE LA SEQUENCE: SEQ ID NO: 2:

```
Met Arg Tyr Ala Ile Val Phe Phe Leu Val Cys Val Ile Thr Leu Gly
  1               5                  10                  15

Glu Ala Leu Lys Cys Val Asn Leu Gln Ala Asn Gly Ile Lys Met Thr
             20                  25                  30

Gln Glu Cys Ala Lys Glu Asp Thr Lys Cys Leu Thr Leu Arg Ser Leu
         35                  40                  45

Lys Lys Thr Leu Lys Phe Cys Ala Ser Gly Arg Thr Cys Thr Thr Met
     50                  55                  60

Lys Ile Met Ser Leu Pro Gly Glu Gln Ile Thr Cys Cys Glu Gly Asn
 65                  70                  75                  80

Met Cys Asn Ala
```

(2) INFORMATION POUR LA SEQ ID NO: 3:

(i) CARACTERISTIQUES DE LA SEQUENCE:

(A) LONGUEUR: 66 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 3:

```
Leu Lys Cys Val Asn Leu Gln Ala Asn Gly Ile Lys Met Thr Gln Glu
1               5                   10                  15

Cys Ala Lys Glu Asp Thr Lys Cys Leu Thr Leu Arg Ser Leu Lys Lys
            20                  25                  30

Thr Leu Lys Phe Cys Ala Ser Gly Arg Thr Cys Thr Thr Met Lys Ile
        35                  40                  45

Met Ser Leu Pro Gly Glu Gln Ile Thr Cys Cys Glu Gly Asn Met Cys
    50                  55                  60

Asn Ala
65
```

(2) INFORMATION POUR LA SEQ ID NO: 4:

(i) CARACTERISTIQUES DE LA SEQUENCE:

(A) LONGUEUR: 66 acides aminés
(B) TYPE: acide aminé
(C) NOMBRE DE BRINS: simple
(D) CONFIGURATION: linéaire

(ii) TYPE DE MOLECULE: peptide

(xi) DESCRIPTION DE LA SEQUENCE: SEQ ID NO: 4:

```
Leu Lys Cys Val Asn Leu Gln Ala Asn Gly Ile Lys Met Thr Gln Glu
1               5                   10                  15

Cys Ala Lys Glu Asp Asn Lys Cys Leu Thr Leu Arg Ser Leu Lys Lys
            20                  25                  30

Thr Leu Lys Phe Cys Ala Ser Asp Arg Ile Cys Lys Thr Met Lys Ile
        35                  40                  45

Met Ser Leu Pro Gly Glu Lys Ile Thr Cys Cys Glu Gly Asn Met Cys
    50                  55                  60

Asn Ala
65
```

(2) INFORMATION POUR LA SEQ ID NO: 5:

(i) CARACTERISTIQUES DE LA SEQUENCE:

(A) LONGUEUR: 66 acides aminés
(B) TYPE: acide aminé
(D) CONFIGURATION: linéaire

(ii) TYPE DE MOLECULE: peptide

(xi) DESCRIPTION DE LA SEQUENCE: SEQ ID NO: 5:

```
Leu Lys Cys Val Asn Leu Gln Ala Asn Gly Val Lys Met Thr Gln Glu
1               5                   10                  15

Cys Ala Lys Glu Asp Thr Lys Cys Leu Thr Leu Arg Ser Leu Lys Lys
            20                  25                  30

Thr Leu Lys Phe Cys Ala Ser Asp Arg Ile Cys Lys Thr Met Lys Ile
        35                  40                  45

Ala Ser Leu Pro Gly Glu Gln Ile Thr Cys Cys Glu Gly Asn Met Cys
    50                  55                  60

Asn Ala
65
```

(2) INFORMATION POUR LA SEQ ID NO: 6:

(i) CARACTERISTIQUES DE LA SEQUENCE:

(A) LONGUEUR: 37 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..37
(D) AUTRES RENSEIGNEMENTS: /note= "partie analysee de xenoxine-1, alkylee"

(xi) DESCRIPTION DE LA SEQUENCE: SEQ ID NO: 6:

```
Leu Lys Cys Val Asn Leu Gln Ala Asn Gly Ile Lys Met Thr Gln Glu
1               5                   10                  15

Cys Ala Lys Glu Asp Thr Lys Cys Leu Thr Leu Arg Ser Leu Lys Lys
            20                  25                  30

Thr Leu Lys Phe Cys
        35
```

(2) INFORMATION POUR LA SEQ ID NO: 7:

(i) CARACTERISTIQUES DE LA SEQUENCE:

(A) LONGUEUR: 43 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..43
(D) AUTRES RENSEIGNEMENTS: /note= "xenoxine-1, alkylee fragment CNBR1(1)"

(xi) DESCRIPTION DE LA SEQUENCE: SEQ ID NO: 7:

```
Leu Lys Cys Val Asn Leu Gln Ala Asn Gly Ile Lys Xaa Xaa Gln Glu
1               5                   10                  15

Cys Ala Lys Glu Asp Thr Lys Cys Leu Thr Leu Arg Ser Leu Lys Lys
            20                  25                  30

Thr Leu Lys Phe Cys Ala Xaa Xaa Xaa Thr Cys
        35                  40
```

(2) INFORMATION POUR LA SEQ ID NO: 8:

(i) CARACTERISTIQUES DE LA SEQUENCE:

(A) LONGUEUR: 31 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..31
(D) AUTRES RENSEIGNEMENTS: /note= "xenoxine-1, alkylee fragment CNBR1(2)"

(xi) DESCRIPTION DE LA SEQUENCE: SEQ ID NO: 8:

```
Thr Gln Glu Cys Ala Lys Glu Asp Thr Lys Cys Leu Thr Xaa Arg Ser
1               5                   10                  15

Leu Lys Lys Thr Leu Lys Phe Cys Ala Xaa Gly Arg Thr Cys Thr
            20                  25                  30
```

(2) INFORMATION POUR LA SEQ ID NO: 9:

(i) CARACTERISTIQUES DE LA SEQUENCE:

(A) LONGUEUR: 8 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..8
(D) AUTRES RENSEIGNEMENTS: /note= "xenoxine-1, alkylee fragment CL1(1)"

(xi) DESCRIPTION DE LA SEQUENCE: SEQ ID NO: 9:

```
Leu Lys Cys Val Asn Leu Gln Ala
1               5
```

(2) INFORMATION POUR LA SEQ ID NO: 10:

(i) CARACTERISTIQUES DE LA SEQUENCE:

(A) LONGUEUR: 12 acides aminés
(B) TYPE: acide aminé
(C) NOMBRE DE BRINS: simple
(D) CONFIGURATION: linéaire

(ii) TYPE DE MOLECULE: peptide
(ix) CARACTERISTIQUE ADDITIONELLE:

(A) NOM/CLE: Peptide
(B) EMPLACEMENT: 1..12
(D) AUTRES RENSEIGNEMENTS: /note= "xenoxine-1, alkylee fragment CL1(2)"

(xi) DESCRIPTION DE LA SEQUENCE: SEQ ID NO: 10:

```
Ser Leu Lys Lys Thr Leu Lys Phe Cys Ala Ser Gly
1               5                   10
```

(2) INFORMATION POUR LA SEQ ID NO: 11:

(i) CARACTERISTIQUES DE LA SEQUENCE:

(A) LONGUEUR: 6 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..6
(D) AUTRES RENSEIGNEMENTS: /note= "xenoxine-1, fragment T1(1)"

(xi) DESCRIPTION DE LA SEQUENCE: SEQ ID NO: 11:

```
Thr Xaa Thr Thr Met Lys
1               5
```

(2) INFORMATION POUR LA SEQ ID NO: 12:

(i) CARACTERISTIQUES DE LA SEQUENCE:

(A) LONGUEUR: 19 acides aminés
(B) TYPE: acide aminé
(C) NOMBRE DE BRINS: simple
(D) CONFIGURATION: linéaire

(il) TYPE DE MOLECULE: peptide

(ix) CARACTERISTIQUE ADDITIONELLE:

(A) NOM/CLE: Peptide
(B) EMPLACEMENT: 1..19
(D) AUTRES RENSEIGNEMENTS: /note= "xenoxine-1, fragment T1(2)"

(xi) DESCRIPTION DE LA SEQUENCE: SEQ ID NO: 12:

```
Ile Met Ser Leu Pro Gly Glu Gln Ile Thr Xaa Xaa Glu Gly Asn Met
1               5                   10                  15

Xaa Asn Ala
```

(2) INFORMATION POUR LA SEQ ID NO: 13:

(i) CARACTERISTIQUES DE LA SEQUENCE:

(A) LONGUEUR: 13 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..13
(D) AUTRES RENSEIGNEMENTS: /note= "xenoxine-1, alkylee fragment CNBR2(1)"

(xi) DESCRIPTION DE LA SEQUENCE: SEQ ID NO: 13:

```
Ser Leu Pro Gly Glu Gln Ile Thr Cys Cys Glu Gly Asn
1               5                   10
```

(2) INFORMATION POUR LA SEQ ID NO: 14:

(i) CARACTERISTIQUES DE LA SEQUENCE:

(A) LONGUEUR: 16 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..16
(D) AUTRES RENSEIGNEMENTS: /note= "xenoxine-1, alkylee fragment CNBR2(2)"

(xi) DESCRIPTION DE LA SEQUENCE: SEQ ID NO: 14:

```
Lys Ile Met Ser Leu Pro Gly Glu Gln Ile Thr Cys Cys Glu Gly Asn
1               5                   10                  15
```

(2) INFORMATION POUR LA SEQ ID NO: 15:

(i) CARACTERISTIQUES DE LA SEQUENCE:

(A) LONGUEUR: 18 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..18
(D) AUTRES RENSEIGNEMENTS: /note= "xenoxine-1, alkylee fragment CL2"

(xi) DESCRIPTION DE LA SEQUENCE: SEQ ID NO: 15:

```
Ile Met Ser Leu Pro Gly Glu Gln Ile Thr Cys Cys Glu Gly Asn Met
1               5                   10                  15

Cys Asn
```

(2) INFORMATION POUR LA SEQ ID NO: 16:

(i) CARACTERISTIQUES DE LA SEQUENCE:

(A) LONGUEUR: 56 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..56
(D) AUTRES RENSEIGNEMENTS: /note= "partie analysee de xenoxine-2, alkylee"

(xi) DESCRIPTION DE LA SEQUENCE: SEQ ID NO: 16:

```
Leu Lys Cys Val Asn Leu Gln Ala Asn Gly Ile Lys Met Thr Gln Glu
1               5                   10                  15

Cys Ala Lys Glu Asp Asn Lys Cys Leu Thr Leu Arg Ser Leu Lys Lys
            20                  25                  30

Thr Leu Lys Phe Cys Ala Xaa Asp Arg Ile Cys Lys Thr Met Lys Ile
        35                  40                  45

Met Ser Leu Pro Gly Glu Xaa Ile
    50                  55
```

(2) INFORMATION POUR LA SEQ ID NO: 17:

(i) CARACTERISTIQUES DE LA SEQUENCE:

(A) LONGUEUR: 47 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..47
(D) AUTRES RENSEIGNEMENTS: /note= "partie analysee de xenoxine-2"

(xi) DESCRIPTION DE LA SEQUENCE: SEQ ID NO: 17:

```
Leu Lys Xaa Val Asn Leu Gln Ala Asn Gly Ile Lys Met Thr Gln Glu
1               5                   10                  15

Xaa Ala Lys Glu Asp Asn Lys Xaa Leu Thr Leu Arg Ser Leu Lys Lys
            20                  25                  30

Thr Leu Lys Phe Cys Ala Ser Asp Xaa Ile Xaa Lys Thr Met Lys
        35                  40                  45
```

(2) INFORMATION POUR LA SEQ ID NO: 18:

(i) CARACTERISTIQUES DE LA SEQUENCE:

(A) LONGUEUR: 11 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..11
(D) AUTRES RENSEIGNEMENTS: /note= "xenoxine-2, alkylee fragment CL3"

(xi) DESCRIPTION DE LA SEQUENCE: SEQ ID NO: 18:

```
Ile Thr Cys Cys Glu Gly Asn Met Cys Asn Ala
1               5                   10
```

(2) INFORMATION POUR LA SEQ ID NO: 19:

(i) CARACTERISTIQUES DE LA SEQUENCE:

(A) LONGUEUR: 8 acides aminés
(B) TYPE: acide aminé
(C) NOMBRE DE BRINS: Simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..8

(D) AUTRES RENSEIGNEMENTS: /note= "xenoxine-2, fragment T3"

(xi) DESCRIPTION DE LA SEQUENCE: SEQ ID NO: 19:

```
Ile Met Ser Leu Pro Gly Glu Lys
1               5
```

(2) INFORMATION POUR LA SEQ ID NO: 20:

(i) CARACTERISTIQUES DE LA SEQUENCE:

(A) LONGUEUR: 20 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..20
(D) AUTRES RENSEIGNEMENTS: /note= "partie analysee de xenoxine-3, alkylee(1)"

(xi) DESCRIPTION DE LA SEQUENCE: SEQ ID NO: 20:

```
Leu Xaa Cys Val Asn Leu Gln Ala Asn Gly Xaa Lys Met Xaa Gln Glu
1               5                   10                  15

Cys Xaa Lys Glu
            20
```

(2) INFORMATION POUR LA SEQ ID NO: 21:

(i) CARACTERISTIQUES DE LA SEQUENCE:

(A) LONGUEUR: 20 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..20
(D) AUTRES RENSEIGNEMENTS: /note= "partie analysée de xenoxine-3, alkylee(2)"

(xi) DESCRIPTION DE LA SEQUENCE: SEQ ID NO: 21:

```
Lys Cys Val Asn Leu Gln Ala Asn Gly Val Lys Met Thr Gln Glu Cys
1               5                   10                  15

Ala Lys Glu Asp
            20
```

(2) INFORMATION POUR LA SEQ ID NO: 22:

(i) CARACTERISTIQUES DE LA SEQUENCE:

(A) LONGUEUR: 20 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..20
(D) AUTRES RENSEIGNEMENTS: /note= "partie analysée de xenoxine 3, alkylee(3)"

(xi) DESCRIPTION DE LA SEQUENCE: SEQ ID NO: 22:

```
Cys Val Asn Leu Gln Ala Asn Gly Val Lys Met Thr Gln Glu Cys Ala
1               5                   10                  15

Lys Glu Asp Thr
            20
```

(2) INFORMATION POUR LA SEQ ID NO: 23:

(i) CARACTERISTIQUES DE LA SEQUENCE:

(A) LONGUEUR: 6 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..6
(D) AUTRES RENSEIGNEMENTS: /note= "xenoxine-3, alkylee fragment T4"

(xi) DESCRIPTION DE LA SEQUENCE: SEQ ID NO: 23:

```
Phe Cys Ala Ser Asp Arg
1               5
```

(2) INFORMATION POUR LA SEQ ID NO: 24:

(i) CARACTERISTIQUES DE LA SEQUENCE:

(A) LONGUEUR: 19 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..19
(D) AUTRES RENSEIGNEMENTS: /note= "xenoxine-3, alkylee fragment T5"

(xi) DESCRIPTION DE LA SEQUENCE: SEQ ID NO: 24:

```
Ile Ala Ser Leu Pro Gly Glu Gln Ile Thr Cys Cys Glu Gly Asn Met
1               5                   10                  15

Cys Asn Ala
```

(2) INFORMATION POUR LA SEQ ID NO: 25:

(i) CARACTERISTIQUES DE LA SEQUENCE:

(A) LONGUEUR: 19 acides aminés
(B) TYPE: acide aminé
(C) NOMBRE DE BRINS: simple
(D) CONFIGURATION: linéaire

(ii) TYPE DE MOLECULE: peptide

(ix) CARACTERISTIQUE ADDITIONELLE:

(A) NOM/CLE: Peptide
(B) EMPLACEMENT: 1..19
(D) AUTRES RENSEIGNEMENTS: /note= "xenoxine-3, alkylee fragment T6"

(xi) DESCRIPTION DE LA SEQUENCE: SEQ ID NO: 25:

```
Ile Ala Ser Leu Pro Gly Glu Gln Ile Thr Xaa Xaa Glu Gly Asn Met
1               5                   10                  15

Xaa Asn Ala
```

(2) INFORMATION POUR LA SEQ ID NO: 26:

(i) CARACTERISTIQUES DE LA SEQUENCE:

(A) LONGUEUR: 28 paires de bases
(B) TYPE: acide nucléique

(C) NOMBRE DE BRINS: simple
(D) CONFIGURATION: linéaire

(ii) TYPE DE MOLECULE: ADNc

(ix) CARACTERISTIQUE ADDITIONELLE:

(A) NOM/CLE: modified_base
(B) EMPLACEMENT: 20
(D) AUTRES RENSEIGNEMENTS: /mod_base= i

(ix) CARACTERISTIQUE ADDITIONELLE:

(A) NOM/CLE: misc_feature
(B) EMPLACEMENT: 1..28
(D) AUTRES RENSEIGNEMENTS: /note= "oligonucleotide OTG3278"

(xi) DESCRIPTION DE LA SEQUENCE: SEQ ID NO: 26:

```
ATGTCGACCA RGARTGYGCN AARGARGA                                    28
```

(2) INFORMATION POUR LA SEQ ID NO: 27:

(i) CARACTERISTIQUES DE LA SEQUENCE:

(A) LONGUEUR: 28 paires de bases
(B) TYPE: acide nucléique
(C) NOMBRE DE BRINS: simple
(D) CONFIGURATION: linéaire

(ii) TYPE DE MOLECULE: ADNc

(ix) CARACTERISTIQUE ADDITIONELLE:

(A) NOM/CLE: modified_base
(B) EMPLACEMENT: 17
(D) AUTRES RENSEIGNEMENTS: /mod_base= i

(ix) CARACTERISTIQUE ADDITIONELLE:

(A) NOM/CLE: misc_feature
(B) EMPLACEMENT: 1..28
(D) AUTRES RENSEIGNEMENTS: /note= "oligonucleotide OTG3279"

(xi) DESCRIPTION DE LA SEQUENCE: SEQ ID NO: 27:

```
ATAAGCTTCA CATRTTNCCY TCRCARCA                                    28
```

(2) INFORMATION POUR LA SEQ ID NO: 28:

(i) CARACTERISTIQUES DE LA SEQUENCE:

(A) LONGUEUR: 149 paires de bases
(B) TYPE: acide nucléique

(C) NOMBRE DE BRINS: simple
(D) CONFIGURATION: linéaire

(ii) TYPE DE MOLECULE: ADNc

(ix) CARACTERISTIQUE ADDITIONELLE:

(A) NOM/CLE: misc_feature
(B) EMPLACEMENT: 1..149
(D) AUTRES RENSEIGNEMENTS: /product= "PCR1" /note= "fragment d'ADNc-PCR utilise pour le criblage decrit dans l'exemple 2.5."

(xi) DESCRIPTION DE LA SEQUENCE: SEQ ID NO: 28:

```
CAGGAGTGTG CGAAGGAGGA TAACAAATGC TTAACATTAA GATCGTTAAA AAAAACTTTA    60
AAGTTTTGTG CCTCTGATCG AATATGTAAG ACTATGAAAA TAATGTCTCT GCCTGGGGAA   120
AAGATTACAT GCTGCGAAGG CAACATGTG                                     149
```